# EUROPEAN PATENT SPECIFICATION

(11) **EP 3 821 011 B1**
(45) Date of publication and mention of the grant of the patent: **26.04.2023**
(21) Application number: 19834769.2
(22) Date of filing: 10.07.2019
(51) Int. Cl.: C12N 15/10, C12Q 1/6855, C40B 40/08

(54) **TRANSPOSOME ENABLED DNA/RNA-SEQUENCING (TED RNA-SEQ)**
TRANSPOSOMENAKTIVIERTE DNA-/RNA-SEQUENZIERUNG (TED RNA-SEQ)
SÉQUENÇAGE D'ADN/ARN ACTIVÉ PAR TRANSPOSOME (SÉQ-ARN TED)

(30) Priority: 11.07.2018 US 201862696681 P
(43) Date of publication of application: 19.05.2021
(73) Proprietor: Cygnus Biosciences (Beijing) Co., Ltd., Beijing 100176 (CN)
(72) Inventor: WU, Yalei, Foster City, California 94404 (US); LEE, Wai Ho, San Francisco, California 94132 (US); ZHENG, Genhua, Fremont, California 94539 (US); LAO, Kai Qin, Pleasanton, California 94566 (US)
(74) Representative: Gille Hrabal Partnerschaftsgesellschaft mbB Patentanwälte
(86) International application number: PCT/US2019/041121
(87) International publication number: WO 2020/014303

(56) References cited:
- WO-A1-2017/048993
- WO-A1-2018/027048
- WO-A2-2015/168161
- US-A1- 2015 368 638
- US-A1- 2015 376 608
- US-A1- 2017 044 525
- US-A1- 2018 016 572
- US-A1- 2018 171 401
- US-A1- 2018 179 579
- DI LIN ET AL: "RNA sequencing by direct tagmentation of RNA/DNA hybrids", PROCEEDINGS OF THE NATIONAL ACADEMY OF SCIENCES, vol. 117, no. 6, 11 February 2020 (2020-02-11), pages 2886-2893, XP055866200, ISSN: 0027-8424, DOI: 10.1073/pnas.1919800117 Retrieved from the Internet: URL:https://www.ncbi.nlm.nih.gov/pmc/artic les/PMC7022195/pdf/pnas.201919800.pdf>

## Description

### CROSS REFERENCE TO RELATED APPLICATIONS

The present application claims priority to U.S. Provisional Application Serial Number 62/696,681, filed July 11, 2018.

### SEQUENCE LISTING

The instant application contains a Sequence Listing which has been submitted electronically in ASCII format and is hereby incorporated by reference in its entirety. Said ASCII copy, created on July 4, 2019, is named 53081-703_601_SL.txt and is 958 bytes in size.

### BACKGROUND

Transposase-mediated fragmentation and tagging of nucleic acid molecules can be used in the preparation of nucleic acid molecules, for example in the generation of sequencing libraries. Tagmentation offers advantages of simplified sample preparation and work-flow in which nucleic acid molecules to be sequenced can be fragmented and tagged in a single reaction. These resulting nucleic acid molecules can be further amplified and/or subjected to sequence specific selection prior to sequencing. Sequence information of nucleic acid molecules can be used to identify sequence variants for diagnostic, therapeutic, forensic, and many other applications.

Simple and rapid sample preparation can be useful for analyzing nucleic acid molecule samples. Nucleic acid molecule samples can be processed in parallel or in bulk, for example, in a high-throughput multiplexing. However, the existing methods of preparing nucleic acid samples often suffer from a number of drawbacks. Amongst them are the time and amount of handling to obtain a nucleic acid library from RNA or transcripts of interest and lack of full length coverage of long nucleic acid molecules in the sample.
WO2018/027048 describes methods and compositions for preparing high-throughput cDNA sequencing libraries. WO2017/048993 describes methods of preparing a next generation sequencing (NGS) library from a ribonucleic acid (RNA) sample.

### SUMMARY

In view of the foregoing, there is a need for improved methods for processing nucleic acid molecules. The methods, compositions, reaction mixtures, and kits provided herein address this need, and provide additional advantages as well. The methods, compositions, reaction mixtures, and kits may comprise a composition of a transposome having a polypeptide transposase and a nucleic acid comprising a mosaic end and a first tag, a reverse transcriptase, and an oligonucleotide comprising a mosaic end and a second tag. A sample comprising a nucleic acid molecule is contacted with the composition to generate a DNA/RNA hybrid molecule having the first or the second tags at the 5' ends and a nucleic acid library that is used for further processing and sequencing.
Use of a composition for tagging of a ribonucleic acid/deoxyribonucleic acid (RNA/DNA) hybrid molecule is the first aspect of the present invention and is provided in claim 1. Preferred embodiments are provided in the dependent claims. Any embodiments of the disclosure below which are not encompassed by the claims are provided for reference only.

Provided herein are compositions comprising a transposome having a polypeptide transposase and a nucleic acid comprising a mosaic end and a first tag, a reverse transcriptase, an oligonucleotide comprising a mosaic end and a second tag. In some instances, the first tag and the second tag share a common nucleic acid sequence. In some instances, the first tag and the second tag comprise distinct nucleic acid sequences. In some instances, the composition comprises a transposome having a polypeptide transposase and a nucleic acid comprising a mosaic end and a second tag. In some instances, the composition further comprises a transposome having a polypeptide transposase and a nucleic acid comprising a mosaic end and a first tag. In some instances, the nucleic acid comprising a mosaic end and a first tag further comprises a tag that identifies a reaction batch. In some instances, the tag that identifies a reaction batch comprises a UMI. In some instances, the oligonucleotide comprises a poly-T segment. In some instances, the oligonucleotide comprises a gene-specific segment. In some instances, the composition comprises a ribonucleic acid sample. In some instances, the composition comprises a deoxyribonucleic acid sample. In some instances, the composition comprises a ribonucleic acid/deoxyribonucleic acid (RNA/DNA) hybrid molecule. In some instances, the composition comprises a template switching oligonucleotide (TSO). In some instances, the TSO comprises a first tag and a mosaic end. In some instances, the composition comprises a protease. In some instances, the protease is active at a temperature no more than 65°C and inactive at a temperature greater than 65°C. In some instances, the composition comprises a polymerase. In some instances, the polymerase is a DNA polymerase. In some instances, the polymerase is a thermostable polymerase. In some instances, the composition comprises a ligase. In some instances, the composition comprises an enzyme for an amplification reaction of a nucleic acid molecule. In some instances, the composition comprises an oligonucleotide comprising a complementary segment to a portion of the first tag or the second tag. In some instances, the oligonucleotide comprises a sequence adapter. In some instances, the transposase is a Tn transposase, an MuA transposase, or a Vibhar transposase. In some instances, the Tn transposase is selected from Tn3, Tn5, Tn7, and Tn10. In some instances, the composition comprises a dual-end tagged RNA/DNA hybrid molecule comprising 5' overhang tags at 5' ends, wherein the individual 5' overhang tag comprises a first tag or a second tag. In some instances, the composition comprises a blunt ended DNA/RNA hybrid molecule comprising complementary segments to the 5' overhang tags from 3' recessed ends of the dual-end tagged RNA/DNA hybrid molecule. In some instances, the composition comprises the blunt ended DNA/RNA hybrid molecules comprising sequence adapters at ends of the blunt-ended DNA/RNA hybrid molecules. In some instances, the composition comprises a sequence library comprising a plurality of blunt ended DNA molecules comprising sequence adapters at ends of the blunt-ended DNA molecules.

Provided herein are methods comprising contacting a sample to a composition comprising a transposome having a transposase polypeptide and a nucleic acid comprising a mosaic end and a first tag, a reverse transcriptase, an oligonucleotide comprising a mosaic end and a second tag. In some instances, the method comprises adding a protease to the composition to inactivate the transposase polypeptide. In some instances, the protease is active at a temperature no more than 65°C and inactive at a temperature greater than 65°C. In some instances, the method comprises adding a polymerase to the sample and the composition. In some instances, the polymerase is a DNA polymerase. In some instances, the polymerase is a thermostable polymerase. In some instances, the method comprises adding an oligonucleotide comprising a sequence adapter to perform an amplification reaction and to generate a sequence library comprising amplification products. In some instances, the first tag and the second tag comprise distinct nucleic acid sequences. In some instances, the method comprises contacting the sample to a transpososome having a transposase polypeptide and a nucleic acid comprising a mosaic end and a second tag. In some instances, the method comprises the first tag and the second tag share a common nucleic acid sequence. In some instances, the method comprises the first tag and the second tag comprise distinct nucleic acid sequences. In some instances, the nucleic acid comprising a mosaic end and a first tag further comprises a tag that identifies a reaction batch. In some instances, the method comprises In some instances, the nucleic acid comprising a mosaic end and a second tag further comprises a tag that identifies a reaction batch. In some instances, the tag that identifies a reaction batch comprises a UMI. In some instances, the oligonucleotide comprises a poly-T segment. In some instances, the oligonucleotide comprises a gene-specific segment. In some instances, the sample comprises a ribonucleic acid sample. In some instances, the sample comprises a deoxyribonucleic acid sample. In some instances, the contacting generates a ribonucleic acid/deoxyribonucleic acid (RNA/DNA) hybrid molecule. In some instances, the composition comprises a template switching oligonucleotide (TSO). In some instances, the TSO comprises a first tag and a mosaic end. In some instances, the oligonucleotide comprising a sequence adapter comprises a cell barcode and a unique molecular identifier (UMI) and is bound to a bead. In some instances, the transposase polypeptide is a Tn transposase, an MuA transposase, or a Vibhar transposase. In some instances, the Tn transposase is selected from Tn3, Tn5, Tn7, and Tn10. In some instances, contacting generates a dual-end tagged RNA/DNA hybrid molecule comprising 5' overhang tags at 5' ends, wherein the individual 5' overhang tag comprises a first tag or a second tag. In some instances, the method generates a blunt ended DNA/RNA hybrid molecule comprising complementary segments to the 5' overhang tags from 3' recessed ends of the dual-end tagged RNA/DNA hybrid molecule. In some instances, the method generates the blunt ended DNA/RNA hybrid molecules comprising sequence adapters at ends of the blunt-ended DNA/RNA hybrid molecules. In some instances, the method generates a sequence library comprising a plurality of blunt ended DNA molecules comprising sequence adapters at ends of the blunt-ended DNA molecules.

Provided herein are methods generating a cDNA library in a single chamber, comprising (a) reverse-transcribing an RNA sample in a sample; (b) introducing complementary cleavage sites into the DNA/RNA hybrid molecule; (c) attaching at cleavage site 5' ends a population of 5' overhang tags; (d) synthesizing complementary segments to the 5' overhang tags from 3' recessed ends of the DNA/RNA hybrid molecule to form blunt ended DNA/RNA hybrid molecules; and (e) adding sequence adapters to the ends of the blunt-ended DNA/RNAs hybrid molecules. In some instances, the method comprises extending 3' end of the cDNA using a template switching oligonucleotide (TSO) during step (a). In some instances, the method comprises introducing complementary cleavage sites into a DNA molecule in the sample; attaching at cleavage site 5' ends a population of 5' overhang tags; synthesizing blunt-ended DNA molecules having a second population of 5' overhang tags and a complementary segment to the DNA molecules having the 5' overhang tags; and adding sequence adapters to the ends of the blunt-ended DNA molecules. In some instances, the reverse-transcribing of step (a) is performed using a reverse transcriptase and an oligonucleotide comprising a mosaic end and a second tag. In some instances, the oligonucleotide comprises a poly-T segment. In some instances, the oligonucleotide comprises a gene-specific segment. In some instances, steps (b) and (c) are performed by a transpososome having a transposase polypeptide and a nucleic acid comprising a mosaic end and a first tag. In some instances, the first tag and the second tag share a common nucleic acid sequence. In some instances, the first tag and the second tag comprise distinct nucleic acid sequences. In some instances, the nucleic acid comprising a mosaic end and a first tag further comprises a tag that identifies a reaction batch. In some instances, the tag that identifies a reaction batch comprises a UMI. In some instances, step (d) comprises an extension reaction. In some instances, step (e) comprises a polymerase chain reaction (PCR). In some instances, the transposase polypeptide is inactivated the using a protease. In some instances, the protease is active at a temperature no more than 65°C and inactive at a temperature greater than 65°C. In some instances, steps (d) and (e) comprise adding a polymerase. In some instances, the polymerase is a DNA polymerase. In some instances, the polymerase is a thermostable polymerase. In some instances, step (e) comprises adding an oligonucleotide comprising a sequence adapter to perform an amplification reaction and to generate the cDNA library comprising amplification products. In some instances, the method generates a ribonucleic acid/deoxyribonucleic acid (RNA/DNA) hybrid molecule. In some instances, the TSO comprises a first tag and a mosaic end. In some instances, the method generates a dual-end tagged RNA/DNA hybrid molecule comprising 5' overhang tags at 5' ends, wherein the individual 5' overhang tag comprises a first tag or a second tag. In some instances, the method comprises the method generates a blunt ended DNA/RNA hybrid molecule comprising complementary segments to the 5' overhang tags from 3' recessed ends of the dual-end tagged RNA/DNA hybrid molecule. In some instances, the method generates the blunt ended DNA/RNA hybrid molecules comprising sequence adapters at ends of the blunt-ended DNA/RNA hybrid molecules. In some instances, the method generates a sequence library comprising a plurality of blunt ended DNA molecules comprising sequence adapters at ends of the blunt-ended DNA molecules.

Provided herein are methods of generating a cDNA library having near full length mRNA coverage, comprising dual end tagged batch labeled cDNA segments, wherein the library is generated in no more than 2 hours. In some instances, the cDNA library is generated by contacting a mRNA sample to a composition comprising a transpososome having a transposase polypeptide and a nucleic acid comprising a mosaic end and a first tag, a reverse transcriptase, an oligonucleotide comprising a mosaic end and a second tag. In some instances, the cDNA library is generated by (a) reverse-transcribing an RNA sample in a sample; (b) introducing complementary cleavage sites into the DNA/RNA hybrid molecule; (c) attaching at cleavage site 5' ends a population of 5' overhang tags; and (d) synthesizing complementary segments to the 5' overhang tags from 3' recessed ends of the DNA/RNA hybrid molecule to form blunt ended DNA/RNA hybrid molecules. In some instances, the library is generated in no more than 1 hour. In some instances, the library is generated in no more than 45 minutes. In some instances, the method further comprises adding sequence adapters to the ends of the blunt-ended DNA/RNA hybrid molecules. In some instances, the method takes no more than 10 hours. In some instances, the method takes no more than 8 hours. In some instances, the method takes no more than 6 hours. In some instances, the method takes no more than 4 hours. In some instances, the method takes no more than 2 hours.

Provided herein are methods of generating a cDNA library having near full length mRNA coverage, comprising dual end tagged batch labeled cDNA segments, wherein the library is generated in a single chamber. In some instances, the cDNA library is generated by contacting a mRNA sample to a composition comprising a transpososome having a transposase polypeptide and a nucleic acid comprising a mosaic end and a first tag, a reverse transcriptase, an oligonucleotide comprising a mosaic end and a second tag. In some instances, the cDNA library is generated by (a) reverse-transcribing an RNA sample in a sample; (b) introducing complementary cleavage sites into the DNA/RNA hybrid molecule; (c) attaching at cleavage site 5' ends a population of 5' overhang tags; (d) synthesizing complementary segments to the 5' overhang tags from 3' recessed ends of the DNA/RNA hybrid molecule to form blunt ended DNA/RNA hybrid molecules; and (e) adding sequence adapters to the ends of the blunt-ended DNA/RNA hybrid molecules.

Provide herein are methods for producing a sequencing library from a dual-end tagged RNA-DNA fragment, comprising: (a) contacting an mRNA molecule to a composition comprising (i) a first transposome comprising a transposase polypeptide and a first transposon nucleic acid, (ii) a reverse transcriptase, and (iii) a reverse transcriptase primer comprising a second transposon sequence, wherein contacting generates a double-stranded mRNA-cDNA intermediate comprising a cDNA strand comprising the reverse transcriptase primer and complementary to the mRNA strand, and a plurality of tagged mRNA-cDNA fragments comprising an overhang comprising the first transposon sequence at a 5' end; (b) inactivating the transposase using a protease; (c) performing an extension reaction to the plurality of tagged mRNA-cDNA fragments to generate a plurality of blunt-ended fragments; and (d) amplifying the plurality of blunt-ended fragments using a first primer comprising the first transposon sequence or a portion of the first transposon sequence to generate a sequencing library comprising amplification products, wherein individual amplification products comprise the first transposon sequence or a complement of the first transposon sequence. In some instances, step (a) is performed in a single chamber. In some instances, the composition further comprises a second transposome comprising a transposase and the second transposon sequence. In some instances, the composition further comprises a third transposome comprising the transposase, the first transposon sequence, and the second transposon sequence. In some instances, the first transposon sequence and the second transposon sequence are identical. In some instances, the first transposon sequence and the second transposon sequence are not identical. In some instances, the transposase is a Tn transposase, an MuA transposase, or a Vibhar transposase. In some instances, the Tn transposase is selected from Tn3, Tn5, Tn7, and Tn10. In some instances, the individual amplification products further comprises a sequence adapter. In some instances, the reverse transcriptase primer comprises a poly-T segment. In some instances, the reverse transcriptase primer comprises a complementary segment to a gene of interest. In some instances, the reverse transcriptase primer comprises a cell barcode and a unique molecular identifier (UMI) and wherein the reverse transcriptase primer is bound to a bead. In some instances, the protease is active at a temperature no more than 65°C and inactive at a temperature greater than 65°C. In some instances, the protease degrades the transposase.

Provided herein are methods for producing a sequencing library from a dual-end tagged RNA-DNA fragment, comprising (a) contacting a mRNA strand to a composition comprising (i) a first transposome comprising a transposase and a first transposon sequence, (ii) a second transposome comprising a transposase and a second transposon sequence, (iii) a reverse transcriptase, and (iv) a reverse transcriptase primer comprising the second transposon sequence, wherein contacting generates a double-stranded mRNA-cDNA intermediate comprising a cDNA strand comprising the reverse transcriptase primer and complementary to the mRNA strand, and a plurality of tagged mRNA-cDNA fragments comprising a first overhang comprising the first transposon sequence at a first 5' end and a second overhang comprising the second transposon sequence at a second 5' end; (b) inactivating the transposases using a protease; (c) performing an extension reaction to the plurality of tagged mRNA-cDNA fragments to generate a plurality of blunt-ended fragments; and (d) amplifying the plurality of blunt-ended fragments using a first primer comprising the first transposon sequence or a portion of the first transposon sequence and a second primer comprising the second transposon sequence or a portion of the second transposon sequence to generate a sequencing library comprising amplification products, wherein individual amplification products comprise the first and the second transposon sequences. In some embodiments, step (a) is performed in a single chamber. In some embodiments, the composition further comprises a third transposome comprising the transposase, the first transposon sequence, and the second transposon sequence. In some embodiments, the first transposon sequence and the second transposon sequence are identical. In some embodiments, the first transposon sequence and the second transposon sequence are not identical. In some embodiments, the transposase is a Tn transposase, an MuA transposase, or a Vibhar transposase. In some embodiments, the Tn transposase is selected from Tn3, Tn5, Tn7, and Tn10. In some embodiments, the reverse transcriptase primer comprises a poly-T segment. In some embodiments, the reverse transcriptase primer comprises a complementary segment to a gene of interest. In some embodiments, the reverse transcriptase primer comprises a cell barcode and a unique molecular identifier (UMI) and wherein the reverse transcriptase primer is bound to a bead. In some embodiments, the individual amplification products further comprises a sequence adapter. In some embodiments, the protease is active at a temperature no more than 65°C and inactive at a temperature greater than 65°C. In some embodiments, the protease degrades the transposase.

Provided herein are compositions comprising (a) a double stranded polynucleotide comprising an RNA strand and a DNA strand; and (b) a transposome comprising a transposase and a transposon sequence, wherein the polynucleotide is contacted to the transposome to generate a tagged fragment comprising an overhang comprising the transposon sequence at a 5' end.

Provided herein are compositions comprising (a) a transposome comprising a transposase and a first transposon sequence; (b) a reverse transcriptase; (c) a reverse transcriptase primer comprising a second transposon sequence; and (d) a protease that is active at a temperature no more than 65°C. In some embodiments, the reverse transcriptase primer comprises a poly-T sequence. In some embodiments, the reverse transcriptase primer comprises a complementary segment to a gene of interest. In some embodiments, the reverse transcriptase primer comprises a cell barcode and a unique molecular identifier (UMI) and wherein the reverse transcriptase primer is bound to a bead. In some embodiments, the composition further comprises a second transposome comprising the transposase and a second transposon sequence. In some embodiments, the composition further comprises a third transposome comprising the transposase, the first transposon sequence, and the second transposon sequence. In some embodiments, the first transposon sequence and the second transposon sequence are identical. In some embodiments, the first transposon sequence and the second transposon sequence are not identical. In some embodiments, the protease is inactivated at a temperature greater than 65°C. In some embodiments, the protease degrades the transposase.

Provided herein are methods of generating a gene panel from a sample comprising (a) contacting a double-stranded polynucleotide and a mRNA strand from a sample to a composition comprising (i) a first transposome comprising a transposase and a first transposon sequence, (ii) a reverse transcriptase, and (iii) a reverse transcriptase primer comprising a gene specific sequence and a second transposon sequence, wherein the contacting generates a double-stranded mRNA-cDNA intermediate comprising a cDNA strand comprising the reverse transcriptase primer and complementary to the mRNA strand and a plurality of tagged mRNA-cDNA fragments comprising a first overhang comprising the first transposon sequence at a first 5' end and a second overhang comprising the second transposon sequence at a second 5' end, and a plurality of tagged double-stranded DNA fragments comprising an overhang comprising the first transposon sequence at a first 5' end; (b) adding a first primer comprising a gene specific sequence and the second transposon sequence, and a second primer comprising a complement of the gene specific sequence and the second transposon sequence to contact the plurality of tagged double-stranded DNA fragments to generate a plurality of double-tagged double-stranded DNA fragments comprising an overhang comprising the first transposon sequence at a first 5' end and an overhang comprising the second transposon sequence at a second 5' end; (c) performing an extension reaction to the plurality of tagged mRNA-cDNA fragments and the plurality of tagged a double-stranded DNA fragments to generate a plurality of blunt-ended fragments; and (d) amplifying the plurality of blunt-ended fragments using a first primer comprising the first transposon sequence or a portion of the first transposon sequence and a second primer comprising the second transposon sequence or a portion of the second transposon sequence to generate amplification products, wherein individual amplification products comprise the first and the second transposon sequences and wherein a gene panel comprises the amplification products. In some embodiments, step (a) is performed in a single chamber. In some embodiments, the gene panel is generated from a single chamber reaction in no more than 10 hours. In some embodiments, the gene panel is generated from a single chamber reaction in no more than 8 hours. In some embodiments, the gene panel is generated from a single chamber reaction in no more than 6 hours. In some embodiments, the gene panel is generated from a single chamber reaction in no more than 4 hours. In some embodiments, the gene panel is generated from a single chamber reaction in no more than 2 hours. In some embodiments, the polynucleotide comprises genomic DNA. In some embodiments, the polynucleotide and the mRNA are from a single cell. In some embodiments, the polynucleotide and the mRNA are obtained from a formalin-fixed paraffin-embedded (FFPE) tissue sample. In some embodiments, the polynucleotide and the mRNA are obtained from a frozen tissue sample. In some embodiments, the composition further comprises a second transposome comprising a transposase and the second transposon sequence. In some embodiments, the composition further comprises a third transposome comprising the transposase, the first transposon sequence, and the second transposon sequence. In some embodiments, the first transposon sequence and the second transposon sequence are identical. In some embodiments, the first transposon sequence and the second transposon sequence are not identical. In some embodiments, the transposase is a Tn transposase, an MuA transposase, or a Vibhar transposase. In some embodiments, the Tn transposase is selected from Tn3, Tn5, Tn7, and Tn10. In some embodiments, the reverse transcriptase primer comprises a poly-T segment. In some embodiments, the reverse transcriptase primer comprises a complementary segment to a gene of interest. In some embodiments, the reverse transcriptase primer comprises a cell barcode and a unique molecular identifier (UMI) and wherein the reverse transcriptase primer is bound to a bead. In some embodiments, the individual amplification products further comprises a sequence adapter.

Provided herein are methods of generating a nucleic acid library, comprising (a) contacting a mRNA strand to a composition comprising (i) a first transposome comprising a transposase and a first transposon sequence, (ii) a second transposome comprising a transposase and a second transposon sequence, (iii) a reverse transcriptase, (iv) a reverse transcriptase primer comprising the second transposon sequence, and (v) a template switching oligonucleotide (TSO) comprising the first transposon sequence, wherein the contacting generates a double stranded mRNA-cDNA intermediate comprising a mRNA strand having a TSO at a 5' end and a cDNA strand comprising the reverse transcriptase primer and complementary to the mRNA strand and to the TSO at a 3' end of cDNA strand, and a plurality of tagged mRNA-cDNA fragments comprising a first overhang comprising the first transposon sequence at a first 5' end and a second overhang comprising the second transposon sequence at a second 5' end; (b) inactivating the transposases using a protease; (c) performing an extension reaction to the plurality of tagged mRNA-cDNA fragments to generate a plurality of blunt-ended fragments; and (d) amplifying the plurality of blunt-ended fragments using a first primer comprising the first transposon sequence or a portion of the first transposon sequence and a second primer comprising the second transposon sequence or a portion of the second transposon sequence to generate amplification products, wherein individual amplification products comprise the first and the second transposon sequences.

Provided herein are methods of generating a nucleic acid library, comprising (a) contacting a mRNA strand to a composition comprising (i) a first transposome comprising a transposase and a first transposon sequence, (ii) a reverse transcriptase, (iii) a reverse transcriptase primer comprising the first transposon sequence, and (iv) a template switching oligonucleotide (TSO) comprising the first transposon sequence, wherein the contacting generates a double stranded mRNA-cDNA intermediate comprising a mRNA strand having a TSO at a 5' end and a cDNA strand comprising the reverse transcriptase primer and complementary to the mRNA strand and to the TSO at a 3' end of cDNA strand, and a plurality of tagged mRNA-cDNA fragments comprising a first overhang comprising the first transposon sequence at a first 5' end and a second overhang comprising the first transposon sequence at a second 5' end; (b) inactivating the transposase using a protease; (c) performing an extension reaction to the plurality of tagged mRNA-cDNA fragments to generate a plurality of first blunt-ended fragments; (d) amplifying the plurality of first blunt-ended fragments using a first primer comprising the first transposon sequence or a portion of the first transposon sequence to generate first amplification products, wherein individual first amplification products comprise the first transposon sequence; (e) contacting the first amplification products to a composition comprising a second transposome comprising a transposase and a second transposon sequence to generate a plurality of tagged fragments comprising a first overhang comprising the first transposon sequence at a first 5' end and a second overhang comprising the second transposon sequence at a second 5' end; (f) performing an extension reaction to the plurality of tagged fragments to generate a plurality of second blunt-ended fragments; and (g) amplifying the plurality of second blunt-ended fragments using a second primer comprising the second transposon sequence or a portion of the second transposon sequence, to generate second amplification products wherein individual second amplification products comprise the first and the transposon sequences. In some embodiments, step (a) is performed in a single chamber. In some embodiments, the use of TSO provides full coverage of the 5' end of the mRNA strand. In some embodiments, the gene panel is generated from a single chamber reaction in no more than 4 hours. In some embodiments, mRNA strand is from a single cell. In some embodiments, the mRNA strand is obtained from a formalin-fixed paraffin-embedded (FFPE) tissue sample. In some embodiments, the mRNA strand is obtained from a frozen tissue sample. In some embodiments, the composition further comprises a second transposome comprising a transposase and the second transposon sequence. In some embodiments, the composition further comprises a third transposome comprising the transposase, the first transposon sequence, and the second transposon sequence. In some embodiments, the first transposon sequence and the second transposon sequence are identical. In some embodiments, the first transposon sequence and the second transposon sequence are not identical. In some embodiments, the transposase is a Tn transposase, an MuA transposase, or a Vibhar transposase. In some embodiments, the Tn transposase is selected from Tn3, Tn5, Tn7, and Tn10. In some embodiments, the transposome comprises a dimer of monomers, wherein an individual monomer comprises a transposase complexed with a transposon sequence. In some embodiments, the reverse transcriptase primer comprises a poly-T segment. In some embodiments, the reverse transcriptase primer comprises a complementary segment to a gene of interest. In some embodiments, the reverse transcriptase primer comprises a cell barcode and a unique molecular identifier (UMI) and wherein the reverse transcriptase primer is bound to a bead. In some embodiments, the individual amplification products further comprises a sequence adapter.

Provided herein are reaction mixtures comprising (a) a mRNA strand from a sample; and (b) a composition comprising (i) a first transposome comprising a transposase and a first transposon sequence, (ii) a reverse transcriptase, and (iii) a reverse transcriptase primer comprising a second transposon sequence. In some embodiments, the reaction mixture comprises a double-stranded polynucleotide comprising genomic DNA from the sample. In some embodiments, the composition further comprises (iv) a first primer comprising a gene specific sequence and the second transposon sequence, and (v) a second primer comprising a complement of the gene specific sequence and the second transposon sequence. In some embodiments, the composition further comprises a second transposome comprising a transposase and the second transposon sequence. In some embodiments, the first and the second transposon sequences are identical. In some embodiments, the first and the second transposon sequences are not identical. In some embodiments, the transposase is a Tn transposase, an MuA transposase, or a Vibhar transposase. In some embodiments, the reaction mixture comprises the Tn transposase is selected from Tn3, Tn5, Tn7, and Tn10. In some embodiments, the reverse transcriptase primer comprises a poly-T segment. In some embodiments, the reverse transcriptase primer comprises a complementary segment to a gene of interest. In some embodiments, the reverse transcriptase primer comprises a cell barcode and a unique molecular identifier (UMI) and wherein the reverse transcriptase primer is bound to a bead. In some embodiments, the composition further comprises a template switching oligonucleotide (TSO). In some embodiments, the TSO comprises a first transposon sequence and a mosaic end. In some embodiments, the composition further comprises a protease that is active at a temperature no more than 65°C and inactive at a temperature greater than 65°C.

Provided herein are kits comprising (a) a set of first transposomes, individual first transposomes comprising a first transposon sequence; (b) a set of second transposomes, individual second transposomes comprising a second transposon sequence; (c) a reverse transcriptase; (d) a reverse transcriptase primer comprising the first transposon sequence; and (e) instructions for practicing method described herein.

Provided herein are kits comprising (a) a set of transposomes comprising (i) a homodimer transposase comprising a first transposon sequence, (ii) a homodimer transposase comprising a second transposon sequence, and optionally (iii) a heterodimer transposase comprising the first transposon sequence and the second transposon sequence; (b) a reverse transcriptase; (c) a reverse transcriptase primer comprising the first transposon sequence; and (d) instructions for practicing methods described herein. In some embodiments, the kit further comprises a template switching oligonucleotide (TSO). In some embodiments, the TSO comprises a first transposon sequence and a mosaic end. In some embodiments, the transcriptase primer comprises a poly-T segment. In some embodiments, the kit further comprises a primer comprising a gene specific sequence. In some embodiments, the reverse transcriptase primer comprises a cell barcode and a unique molecular identifier (UMI) and wherein the reverse transcriptase primer is bound to a bead. In some embodiments, the kit further comprises a primer complementary segment to a gene specific sequence. In some embodiments, the kit further comprises a protease that is active at a temperature no more than 65°C and inactive at a temperature greater than 65°C.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments and the accompanying drawings (also "Figure" and "FIG." herein), of which:
**FIG. 1** illustrates an example method of producing a cDNA library of an mRNA molecule from dual-end tagged RNA-DNA hybrid fragments.
**FIG. 2** illustrates an example method of producing a library of nucleic acid molecules from dual-end tagged nucleic acid fragments.
**FIG. 3** illustrates an example method of producing a cDNA library of an mRNA molecule from dual-end tagged RNA-DNA hybrid fragments.
**FIG. 4** illustrates an example method of producing a cDNA library of an mRNA molecule from dual-end tagged RNA-DNA hybrid fragments.
**FIG. 5** illustrates an example method of producing a cDNA library of an mRNA molecule from dual-end tagged RNA-DNA hybrid fragments.

### DETAILED DESCRIPTION

Transposase-mediated fragmentation and tagging of nucleic acid molecules is useful in the preparation of nucleic acid molecules. The transposase-mediated fragmentation and tagging, also referred to as tagmentation or transposition, provide advantages of simplified sample preparation and work-flow in which the nucleic acid molecules of interest can be fragmented and tagged in a single reaction. In a sample comprising RNA molecules, the sample may undergo reverse transcription to generate a double-stranded RNA-cDNA hybrid and tagmentation to generate tagged double-stranded RNA-cDNA hybrid. The reverse transcription and tagmentation can be performed in a single chamber, such as a well, spot on a surface, droplet alone or in an emulsion, or other compartment. The reverse transcription and tagmentation is in some cases performed without further user handling beyond the initial contacting of the nucleic acid molecule to the composition described herein. The tagged nucleic acid molecules may allow for full length or almost full length coverage of the nucleic acid molecule in the nucleic acid library. The tagged nucleic acid molecules can be further amplified or selected for specific sequences prior to sequencing. The further processing steps may utilize the tags on the tagged nucleic acid molecules resulting from the tagmentation to aid in the amplification, selection, sequencing, or other steps. For example, the sequence information of the resulting nucleic acid molecules can be used to identify sequence variants for diagnostic, therapeutic, forensic, and many other applications. The tagmentation process as described herein may offer shorter processing time, less handling, and closer to full length coverage of long nucleic acid molecules in the sample in generating a nucleic acid library.

Described herein are methods, compositions, reaction mixtures, and kits for generating a nucleic acid library. Methods, compositions, reaction mixtures, and kits variously comprise a composition of a transposome having a polypeptide transposase and a nucleic acid comprising a mosaic end and a first tag, a reverse transcriptase, and an oligonucleotide comprising a mosaic end and a second tag, wherein the oligonucleotide may be a reverse transcriptase primer. In some instances, a sample comprising an mRNA molecule may be contacted with the composition to generate a ribonucleic acid-deoxyribonucleic acid (RNA-DNA) hybrid molecule that is then used to generate a dual-end tagged RNA-DNA hybrid having the first or the second tags at the 5' ends. In some instances, the polypeptide transposase is inactivated by a protease after the dual-end tagged RNA-DNA hybrid is generated. The dual-end tagged RNA-DNA hybrid may have 5' overhang fragments comprising the first tag or the second tag. In some instances, the dual-end tagged RNA-DNA hybrid having overhang tags may be subjected to an extension reaction to generate a blunt-ended, dual-end tagged DNA-RNA hybrid comprising complementary segments to the 5' overhang tags from 3' recessed ends of the dual-end tagged RNA/DNA hybrid molecule. In some instances, a sample comprising a double-stranded nucleic acid molecule may be contacted with the composition to generate a dual-end tagged nucleic acid molecule having the first or the second tags at the 5' ends. The dual-end tagged RNA-DNA hybrid or the dual-end tagged nucleic acid molecule may be subjected to amplification and/or selection step to generate a nucleic acid library for further processing and sequencing.

Described herein are compositions comprising a transposome having a polypeptide transposase and a nucleic acid comprising a mosaic end and a first tag, a reverse transcriptase, an oligonucleotide comprising a mosaic end and a second tag.

Described herein are methods comprising contacting a sample to a composition comprising a transposome having a transposase polypeptide and a nucleic acid comprising a mosaic end and a first tag, a reverse transcriptase, an oligonucleotide comprising a mosaic end and a second tag.

Also described herein is a method of generating a cDNA library in a single chamber, comprising at least one of (a) reverse-transcribing an RNA sample in a sample; (b) introducing complementary cleavage sites into the DNA/RNA hybrid molecule; (c) attaching at cleavage site 5' ends a population of 5' overhang tags; and (d) synthesizing complementary segments to the 5' overhang tags from 3' recessed ends of the DNA/RNA hybrid molecule to form blunt ended DNA/RNA hybrid molecules.

Further described herein is a method of generating a cDNA library having near full length mRNA coverage, comprising dual end tagged batch labeled cDNA segments, wherein the library is generated in no more than 2 hours.

Also described herein is a method of generating a cDNA library having near full length mRNA coverage, comprising dual end tagged batch labeled cDNA segments, wherein the library is generated in a single chamber.

Described herein are methods for producing a sequencing library from a dual-end tagged RNA-DNA fragment, comprising contacting an mRNA molecule to a composition comprising at least one of (i) a first transposome comprising a transposase polypeptide and a first transposon nucleic acid, (ii) a reverse transcriptase, and (iii) a reverse transcriptase primer comprising a second transposon nucleic acid, wherein contacting generates a double-stranded mRNA-cDNA intermediate comprising a cDNA strand comprising the reverse transcriptase primer and complementary to the mRNA strand, and a plurality of tagged mRNA-cDNA fragments comprising an overhang comprising the first transposon nucleic acid at a 5' end; inactivating the transposase using a protease; performing an extension reaction to the plurality of tagged mRNA-cDNA fragments to generate a plurality of blunt-ended fragments; and amplifying the plurality of blunt-ended fragments using a first primer comprising the first transposon nucleic acid or a portion of the first transposon nucleic acid to generate a sequencing library comprising amplification products, wherein individual amplification products comprise the first transposon nucleic acid or a complement of the first transposon nucleic acid.

Described herein is a method for producing a sequencing library from a dual-end tagged RNA-DNA fragment, comprising contacting a mRNA strand to a composition comprising (i) a first transposome comprising a transposase and a first transposon nucleic acid, (ii) a second transposome comprising a transposase and a second transposon nucleic acid, (iii) a reverse transcriptase, and (iv) a reverse transcriptase primer comprising the second transposon nucleic acid, wherein contacting generates a double-stranded mRNA-cDNA intermediate comprising a cDNA strand comprising the reverse transcriptase primer and complementary to the mRNA strand, and a plurality of tagged mRNA-cDNA fragments comprising a first overhang comprising the first transposon nucleic acid at a first 5' end and a second overhang comprising the second transposon nucleic acid at a second 5' end; inactivating the transposases using a protease; performing an extension reaction to the plurality of tagged mRNA-cDNA fragments to generate a plurality of blunt-ended fragments; and amplifying the plurality of blunt-ended fragments using a first primer comprising the first transposon nucleic acid or a portion of the first transposon nucleic acid and a second primer comprising the second transposon nucleic acid or a portion of the second transposon nucleic acid to generate a sequencing library comprising amplification products, wherein individual amplification products comprise the first and the second transposon nucleic acids.

Further described herein is a composition comprising a double stranded nucleic acid molecule comprising an RNA strand and a DNA strand; and a transposome comprising a transposase and a transposon nucleic acid, wherein the nucleic acid molecule is contacted to the transposome to generate a tagged fragment comprising an overhang comprising the transposon nucleic acid at a 5' end.

Also described herein is a composition comprising a transposome comprising a transposase and a first transposon nucleic acid; a reverse transcriptase; a reverse transcriptase primer comprising a second transposon nucleic acid; and a protease that is active at a temperature no more than 65°C.

Described herein are methods of generating a gene panel from a sample comprising contacting a double-stranded nucleic acid molecule and a mRNA strand from a sample to a composition comprising at least one of (i) a first transposome comprising a transposase and a first transposon nucleic acid, (ii) a reverse transcriptase, and (iii) a reverse transcriptase primer comprising a gene specific sequence and a second transposon nucleic acid, wherein the contacting generates a double-stranded mRNA-cDNA intermediate comprising a cDNA strand comprising the reverse transcriptase primer and complementary to the mRNA strand and a plurality of tagged mRNA-cDNA fragments comprising a first overhang comprising the first transposon nucleic acid at a first 5' end and a second overhang comprising the second transposon nucleic acid at a second 5' end, and a plurality of tagged double-stranded DNA fragments comprising an overhang comprising the first transposon nucleic acid at a first 5' end; adding a first primer comprising a gene specific sequence and the second transposon nucleic acid, and a second primer comprising a complement of the gene specific sequence and the second transposon nucleic acid to contact the plurality of tagged double-stranded DNA fragments to generate a plurality of double-tagged double-stranded DNA fragments comprising an overhang comprising the first transposon nucleic acid at a first 5' end and an overhang comprising the second transposon nucleic acid at a second 5' end; performing an extension reaction to the plurality of tagged mRNA-cDNA fragments and the plurality of tagged a double-stranded DNA fragments to generate a plurality of blunt-ended fragments; and amplifying the plurality of blunt-ended fragments using a first primer comprising the first transposon nucleic acid or a portion of the first transposon nucleic acid and a second primer comprising the second transposon nucleic acid or a portion of the second transposon nucleic acid to generate amplification products, wherein individual amplification products comprise the first and the second transposon nucleic acids and wherein a gene panel comprises the amplification products.

Further described herein is a method of generating a nucleic acid library, comprising contacting a mRNA strand to a composition comprising at least one of (i) a first transposome comprising a transposase and a first transposon nucleic acid, (ii) a second transposome comprising a transposase and a second transposon nucleic acid, (iii) a reverse transcriptase, (iv) a reverse transcriptase primer comprising the second transposon nucleic acid, and (v) a template switching oligonucleotide (TSO) comprising the first transposon nucleic acid, wherein the contacting generates a double stranded mRNA-cDNA intermediate comprising a mRNA strand having a TSO at a 5' end and a cDNA strand comprising the reverse transcriptase primer and complementary to the mRNA strand and to the TSO at a 3' end of cDNA strand, and a plurality of tagged mRNA-cDNA fragments comprising a first overhang comprising the first transposon nucleic acid at a first 5' end and a second overhang comprising the second transposon nucleic acid at a second 5' end; inactivating the transposases using a protease; performing an extension reaction to the plurality of tagged mRNA-cDNA fragments to generate a plurality of blunt-ended fragments; and amplifying the plurality of blunt-ended fragments using a first primer comprising the first transposon nucleic acid or a portion of the first transposon nucleic acid and a second primer comprising the second transposon nucleic acid or a portion of the second transposon nucleic acid to generate amplification products, wherein individual amplification products comprise the first and the second transposon nucleic acids.

Also described herein are methods of generating a nucleic acid library, comprising at least one of contacting a mRNA strand to a composition comprising (i) a first transposome comprising a transposase and a first transposon nucleic acid, (ii) a reverse transcriptase, (iii) a reverse transcriptase primer comprising the first transposon nucleic acid, and (iv) a template switching oligonucleotide (TSO) comprising the first transposon nucleic acid, wherein the contacting generates a double stranded mRNA-cDNA intermediate comprising a mRNA strand having a TSO at a 5' end and a cDNA strand comprising the reverse transcriptase primer and complementary to the mRNA strand and to the TSO at a 3' end of cDNA strand, and a plurality of tagged mRNA-cDNA fragments comprising a first overhang comprising the first transposon nucleic acid at a first 5' end and a second overhang comprising the first transposon nucleic acid at a second 5' end; inactivating the transposase using a protease; performing an extension reaction to the plurality of tagged mRNA-cDNA fragments to generate a plurality of first blunt-ended fragments; amplifying the plurality of first blunt-ended fragments using a first primer comprising the first transposon nucleic acid or a portion of the first transposon nucleic acid to generate first amplification products, wherein individual first amplification products comprise the first transposon nucleic acid; contacting the first amplification products to a composition comprising a second transposome comprising a transposase and a second transposon nucleic acid to generate a plurality of tagged fragments comprising a first overhang comprising the first transposon nucleic acid at a first 5' end and a second overhang comprising the second transposon nucleic acid at a second 5' end; performing an extension reaction to the plurality of tagged fragments to generate a plurality of second blunt-ended fragments; and amplifying the plurality of second blunt-ended fragments using a second primer comprising the second transposon nucleic acid or a portion of the second transposon nucleic acid, to generate second amplification products wherein individual second amplification products comprise the first and the transposon nucleic acids.

Further described herein are reaction mixtures comprising a mRNA strand from a sample; and a composition comprising at least one of (i) a first transposome comprising a transposase and a first transposon nucleic acid, (ii) a reverse transcriptase, and (iii) a reverse transcriptase primer comprising a second transposon nucleic acid.

Also described herein is a kit comprising a set of first transposomes, individual first transposomes comprising a first transposon nucleic acid; a set of second transposomes, individual second transposomes comprising a second transposon nucleic acid; a reverse transcriptase; a reverse transcriptase primer comprising the first transposon nucleic acid; and instructions for practicing methods as described herein.

Further described herein is a kit comprising: a set of transposomes comprising a homodimer transposase comprising a first transposon nucleic acid, a homodimer transposase comprising a second transposon nucleic acid, and optionally a heterodimer transposase comprising the first transposon nucleic acid and the second transposon nucleic acid; a reverse transcriptase; a reverse transcriptase primer comprising the first transposon nucleic acid; and instructions for practicing methods described herein.

### Sample Preparation

The present disclosure provides methods, compositions, reaction mixtures, kits, and systems for generating a library of nucleic acid molecules. The nucleic acid molecule can be part of a nucleic acid molecule sample. Sometimes nucleic acid molecule is an isolated nucleic acid molecule. Often, the nucleic acid molecule is from a cell-based sample. In some instances, the nucleic acid molecule is from a single cell. In some instances, the nucleic acid molecule is from a formalin-fixed paraffin embedded (FFPE) sample. In some instances, the nucleic acid molecule is from a frozen tissue sample. The nucleic acid molecule may comprise ribonucleic acid The nucleic acid molecule may comprise deoxyribonucleic acid. The methods, compositions, reaction mixtures, kits, and systems described herein can be useful for preparing a library from a nucleic acid molecule such as, but not limited to RNA, mRNA, nucleic acid transcripts, cell-free DNA, and genomic DNA.

Any of a variety of nucleic acid molecules can be characterized by methods of the present disclosure. In some embodiments, the nucleic acid molecule characterized can be an RNA. In some embodiments, the nucleic acid molecule characterized can be an mRNA. In some embodiments, the nucleic acid molecule characterized can be a genomic nucleic acid molecule. In some cases, the genomic nucleic acid molecule can comprise genomic DNA, genomic RNA or a mixture of genomic DNA and genomic RNA. In some cases, the genomic nucleic acid molecule can exhibit high integrity, such as high molecular weight nucleic acid molecules. In some cases, the genomic nucleic acid molecule can be further bound to proteins, such as histones. In some cases, the genomic nucleic acid molecule can be fragmented. In some cases, the nucleic acid molecule can be methylated, such as 5-methylcytosine (e.g., partially or completely). In some cases, the nucleic acid molecule can be unmethylated. In some cases the genomic nucleic acid molecule comprises a chromosome. A chromosome can be any chromosome of eukaryotic cell or a prokaryotic cell. In some embodiments, the nucleic acid molecule characterized is a cell-free nucleic acid molecule. In some embodiments, the cell-free nucleic acid molecule is cell-free DNA (cfDNA) or cell-free RNA (cfRNA). For example, a cell-free nucleic acid molecule can be circulating tumor DNA, circulating tumor RNA, circulating fetal DNA, or circulating fetal RNA.

The nucleic acid molecule can be from intact cells or tissues. The nucleic acid molecule may be from a single cell. In some cases, the nucleic acid molecules can be obtained from bodily fluids, such as blood, saliva, urine, amniotic fluid, plasma, mucous, cerebral spinal fluid, tears, synovial fluid, lymph, lactal duct fluid, and semen, etc. In certain embodiments, the nucleic acid molecules are isolated from fresh tissues. In other cases, the nucleic acid molecule sample can be isolated from frozen tissues. In yet other cases, the nucleic acid molecule sample can be isolated from fixed tissues, such as formalin-fixed paraffin-embedded (FFPE) tissues. Further examples of sources of nucleic acid molecule samples include, but are not limited to, cells dissociated from tissues, blood cells, bacteria, virus, mitochondria, chloroplast, in vitro assembled protein DNA complexes, and neutrophil extracellular traps. In some cases, the nucleic acid molecule can be obtained from a culture of cells, e.g., a cell line. The nucleic acid molecule can be fragmented to yield fragments suitable for downstream assays.

Cell-free nucleic acid molecules include nucleic acid molecules originating from a cell but not directly obtained from a cellular source, such as a tissue sample. Non-limiting examples of sources from which cell-free nucleic acid molecules may originate are normal cells and tissue, abnormal cells and tissue (e.g., diseased cells or tissue, e.g., cancerous cells or tissue), fetal cells and tissue, and pathogens. A cell-free nucleic acid molecule present in a non-cellular source can result from cell death (e.g., apoptosis or necrosis) or cell shedding. Sequence analysis of cell-free nucleic acid molecules can be used to characterize the cell or population of cells from which the cell-free nucleic acid molecule is derived, such as tumor cells (e.g., in cancer detection), fetal cells (e.g., in prenatal diagnostics), cells from transplanted tissue (e.g., in early detection of transplant failure), or a pathogen (e.g., bacteria or virus).

A large range of nucleic acid molecules are compatible with various embodiments of the present disclosure. Nucleic acid molecules can be obtained from a subject, such as any animal or living organism. Non-limiting examples of subjects are mammals, such as humans, non-human primates, rodents such as mice and rats, dogs, cats, pigs, sheep, rabbits and others. In some embodiments, a subject is healthy, and nucleic acid molecules obtained from the subject may not comprise a sequence variant associated with a disease or disorder. In some embodiments, a subject is suspected of having a disease or disorder, and nucleic acid molecules obtained from the subject may comprise a sequence variant associated with the disease or disorder. In some embodiments, a subject is pregnant, and nucleic acid molecules obtained from the subject comprise fetal nucleic acid molecules.

Target nucleic acid molecules variously comprise RNA such as mRNA or viral genomic RNA, or DNA such as genomic DNA. In some embodiments, a nucleic acid molecule or a target nucleic acid molecule is derived from RNA such as mRNA, genomic DNA or another nucleic acid source. RNA such as mRNA, or genomic DNA can be obtained from a cell or tissue sample using various methods and commercial kits available, such as a Qiagen RNeasy Mini Kit or Qiagen DNeasy Tissue Kit. RNA, mRNA, or genomic DNA can be obtained and purified from a sample using any extraction, isolation, and purification method previously described elsewhere herein. Other non-limiting examples of extraction techniques include: (1) organic extraction followed by ethanol precipitation, e.g., using a phenol/chloroform organic reagent (Ausubel et al., 1993), with or without the use of an automated nucleic acid extractor, e.g., the Model 341 DNA Extractor available from Applied Biosystems (Foster City, Calif); (2) stationary phase adsorption methods (U.S. Pat. No. 5,234,809; Walsh et al., 1991); and (3) salt-induced nucleic acid precipitation methods (Miller et al., (1988), such precipitation methods being typically referred to as "salting-out" methods. Another example of nucleic acid isolation and/or purification includes the use of magnetic particles to which nucleic acids can specifically or non-specifically bind, followed by isolation of the beads using a magnet, and washing and eluting the nucleic acids from the beads (see e.g. U.S. Pat. No. 5,705,628). For example, nucleic acids can be isolated and purified using solid phase reversible immobilization (SPRI) beads (Agencourt AMPure XP). In some embodiments, the above isolation methods may be preceded by an enzyme digestion step to help eliminate unwanted protein from the sample, e.g., digestion with proteinase K, or other like proteases. If desired, RNase inhibitors may be added to the lysis buffer. For certain cell or sample types, it may be desirable to add a protein denaturation/digestion step to the protocol. Purification methods may be directed to isolate DNA, RNA, or both. When both DNA and RNA are isolated together during or subsequent to an extraction procedure, further steps may be employed to purify one or both separately from the other. Sub-fractions of extracted nucleic acids can also be generated, for example, purification by size, sequence, or other physical or chemical characteristic. In addition to an initial nucleic acid isolation step, purification of nucleic acids can be performed after any step in the disclosed methods, such as to remove excess or unwanted reagents, reactants, or products. A variety of methods for determining the amount and/or purity of nucleic acids in a sample are available, such as by absorbance (e.g. absorbance of light at 260 nm, 280 nm, and a ratio of these) and detection of a label (e.g. fluorescent dyes and intercalating agents, such as SYBR green, SYBR blue, DAPI, propidium iodide, Hoechst stain, SYBR gold, ethidium bromide).

In some embodiments, a nucleic acid molecule or a target nucleic acid molecule comprises fragmented RNA, fragmented mRNA, fragmented cell-free DNA, or fragmented genomic DNA. In some cases, fragmenting occurs as a result or prior sample processing steps, such as formalin fixation, paraffin embedding, or freezing. In some cases, a nucleic acid molecule is fragmented to yield shorter fragments.

In some embodiments, genomic DNA is fragmented into nucleic acid molecules of shorter lengths. In some embodiments, genomic DNA fragments are approximately uniform in length. In some embodiments, genomic DNA fragments are not approximately uniform in length. In some embodiments, genomic DNA fragments have an average length from about 50 to about 100 nucleotides in length. In some embodiments, genomic DNA fragments have an average length from about 50 and 250 nucleotides in length. In some embodiments, genomic DNA fragments have an average length from about 50 and 500 nucleotides in length. In some embodiments, genomic DNA fragments have an average length from about 50 and 750 nucleotides in length. In some embodiments, genomic DNA fragments have an average length from about 100 and 1000 nucleotides in length.

### Reverse Transcription

Described herein are methods, compositions, reaction mixtures, kits, and systems that comprise use of a reverse transcriptase for reverse transcription of an RNA molecule to generate a complementary DNA (cDNA) to the RNA template, often as part of nucleic acid sequence determination. Generally, a reverse transcriptase has both an RNA-directed DNA polymerase activity and a DNA-directed DNA polymerase activity. The RNA molecule often is an mRNA molecule, rRNA molecule, noncoding RNA molecule, viral genomic RNA molecule, or other RNA molecule. During reverse transcription, a reverse transcriptase primer anneals to the RNA template in order for DNA synthesis to be initiated from the 3' OH of the primer by the reverse transcriptase. In some instances, reverse transcriptase may allow formation of matched DNA/RNA hybrids. The cDNA products of reverse transcription are often used for subsequent cDNA analyses, such as cDNA sequencing, real-time polymerase chain reaction (PCR), and alkaline agarose gel electrophoresis, among others.

A number of reverse transcriptases are compatible with the disclosure herein to generate the cDNA library as disclosed herein. Compatible enzymes include viral reverse transcriptases. In some instances, the reverse transcriptase is derived from a virus, an avian virus, a human virus, a murine virus, a retrovirus, a lentivirus, or a gammateretrovirus. In some cases, the viral reverse transcriptase is derived from a Moloney Murine Leukemia Virus (M-MLV) reverse transcriptase, Rous Sarcoma Virus (RSV) reverse transcriptase, Avian Myeloblastosis Virus (AMV) reverse transcriptase, Myeloblastosis Associated Virus (MAV) reverse transcriptase, Rous Associated Virus (RAV) reverse transcriptase, or Human Immunodeficiency Virus 1 (HIV-1) reverse transcriptase. In some cases, the polymerase of the reverse transcriptase is a DNA polymerase. In some cases, the polymerase of the reverse transcriptase is an RNA-dependent DNA polymerase. In some embodiments, the polymerase of the reverse transcriptase is a DNA-dependent DNA polymerase. In some cases, the viral reverse transcriptase is Moloney Murine Leukemia Virus (M-MLV) reverse transcriptase. In some embodiments, the viral reverse transcriptase is HIV-1 reverse transcriptase. In some instances, the reverse transcriptase is a non-natural reverse transcriptase.

A number of additional reverse transcriptases are consistent with the disclosure herein. Often, the reverse transcriptase comprises at least one domain or sequence derived from a retrovirus (e.g., M-MLV, MLV, AMV) and at least one domain or sequence derived from a bacterium (e.g., extremophile bacterium, Thermus bacterium, T. thermophilus, T. Thermophilus HB8) in any combination thereof. For example, the reverse transcriptase may comprise an M-MLV domain (e.g., an M-MLV DNA polymerase domain) and an extremophile domain (e.g., RNase from an extremophile bacterium); or, the reverse transcriptase may comprise a MLV domain (e.g., an MLV DNA polymerase domain) and a Thermus bacterium domain (e.g., RNase from T. Thermophilus). Often, the reverse transcriptases provided herein comprise at least one domain or sequence derived from a retrovirus (e.g., M-MLV, MLV, AMV) and at least one domain or sequence derived from an archaea (e.g., extremophile archaea, T. litoralis) in any combination. Generally, the DNA polymerase domain (or domains) is derived from a virus. Generally, the RNase domain (or domains) is derived from a bacterium or archaea organism.

The single reverse transcription and tagmentation reaction disclosed herein uses a reverse transcriptase primer (RT primer) to synthesize a cDNA to the RNA template by the reverse transcriptase. RT primers are nucleic acid molecules, typically an oligonucleotide. For reverse transcription of an mRNA molecule, the RT primer hybridizes to the poly A tail at the 3' end of the mRNA molecule, and the reverse transcriptase synthesizes a cDNA molecule complementary to the mRNA template, extending from the 3' OH of the RT primer. ART primer may not be limited to a specific sequence so long as the nucleotide sequence of the primer is complementary (e.g., partially or fully) to that of the template RNA and that can anneal to the template RNA under desired reaction conditions. The RT primer often comprises a poly T segment that can hybridize to a poly A tail found at a 3' end of an mRNA molecule. In some cases, the RT primer is a gene-specific primer having a complementary segment to a gene of interest. In some cases, the RT primer is an oligonucleotide having a random sequence (e.g., a random primer). Sometimes the RT primer comprises at least one of a first tag (N1) or a second tag (N2). In some cases, the RT primer further comprises a mosaic end (ME) linked to the first or the second tag. In some cases, the RT primer is N1ME-T20VN.

In various embodiments described herein, the reverse transcriptase is SuperScript IV reverse transcriptase. Some exemplary reverse transcription reactions using SuperScript IV take place at 50°C for 30 minutes in some embodiments, though variants in incubation temperature, incubation time, or both incubation time and temperature are also contemplated..

The disclosure provided herein may optionally use a template switching oligonucleotide (TSO) to provide a full coverage of the mRNA molecule in the resulting cDNA library. The TSO hybridizes to the 3' end of the cDNA that often comprises a poly-deoxycytidine segment. In some cases, the TSO links to a 5' end of the mRNA molecules. In some cases, the TSO comprises a poly-guanidine ribonucleotide segment. In some cases, the TSO further comprises a mosaic end and a first tag (N1) at a '5 end. In some cases, the TSO comprises a mosaic end and a second tag (N2) at a '5 end. The addition of TSO in the reverse transcription and tagmentation reaction allows for a full length capture of the mRNA molecules. The mRNA-cDNA hybrid molecules having a TSO undergo a tagmentation reaction by a transposome in the same reaction chamber.

In various embodiments disclosed herein, reverse transcription and tagmentation reaction without a TSO provides almost full length coverage of the mRNA molecule in the cDNA library generated by the disclosure herein. In some cases, the almost full length coverage refers to at least 75%, at least 80%, at least 85%, at least 90%, at least 91%, at least 92%, at least 93%, at least 94%, at least 95%, at least 96%, at least 97%, at least 98%, or at least 99% of the full length of the template molecule. In some cases, the almost full length coverage refers to about 75%, 80%, 85%, 90%, 91%, 92%, 93%, 94%, 95%, 96%, 97%, 98%, or 99% of the full length of the template molecule. In some cases, reverse transcription and tagmentation reaction using a TSO provides full length coverage of the mRNA molecule or about 100% of the full length of the template molecule.

### Transposomes

Described herein are methods, compositions, reaction mixtures, kits, and systems using a transposome, also referred to as a transpososome, for tagmentation of a nucleic acid molecule from a sample to generate a nucleic acid library from the tagged nucleic acid molecule. During tagmentation or transposition, an overhang fragment comprising a first transposon nucleic acid at a 5' end is formed by contacting the double-stranded nucleic acid molecule with a transposome. Often, the transposome is a complex of a transposase having or bound to a transposon nucleic acid. The transposon nucleic acid comprises a transposon element, which can also be referred to as a transposase element. Transposases refer to polypeptides capable of complexing with at least one transposon nucleic acid and catalyzing insertion or transposition of the transposon nucleic acid into a nucleic acid molecule to yield a modified or "tagged" nucleic acid molecule. Transposases, generally, can catalyze insertion or transposition of the transposon nucleic acid to a nucleic acid molecule by a cut and paste mechanism or a replicative transposition mechanism. In some cases, the transposome is a dimer of monomers, individual monomers comprising a transposase and a transposon nucleic acid. The transposome dimer can be a homodimer or a heterodimer. In some instances, the transposon nucleic acids of a homodimer are the same transposon nucleic acid. In some instances, the transposon nucleic acids of a heterodimer are different transposon nucleic acids. Sometimes the transposition reaction can be facilitated and/or triggered by addition of one or more cations. In some cases, the cations are divalent cations such as, for example, Ca²⁺, Mg²⁺ and Mn²⁺.

Transposase described herein refers to enzymes that can catalyze insertion of a transposon nucleic acid into a nucleic acid molecule to generate a tagged nucleic acid molecule. The transposase can be any naturally occurring transposase or an engineered (e.g., mutated or mutant transposase). Transposases refer to enzymes capable of complexing with at least one transposon nucleic acid and catalyzing insertion or transposition of the transposon nucleic acid into a target nucleic acid molecule. The transposases can be of prokaryotic or eukaryotic origin. Example transposases include, but are not limited to, integrases, HERMES, and HIV integrases. Non-limiting examples of transposases which can be used in embodiments herein include Tn transposases (e.g. Tn3, Tn5, Tn7, Tn10, Tn552, Tn903), MuA transposases, Vibhar transposases (e.g. from *Vibrio harveyi*), Ac-Ds, Ascot-1, Bs1, Cin4, Copia, En/Spm, F element, hobo, Hsmarl, Hsmar2, IN (HIV), IS1, IS2, IS3, IS4, IS5, IS6, IS10, IS21, IS30, IS50, IS51, IS150, IS256, IS407, IS427, IS630, IS903, IS911, IS982, IS1031, ISL2, L1, Mariner, P element, Tam3, Tc1, Tc3, Tel, THE-1, Tn/O, TnA, Tol1, Tol2, TnlO, Tyl, any prokaryotic transposase, or any transposase related to and/or derived from those provided herein. In some embodiments, a subject method utilizes a Tn transposase, an MuA transposase, or a Vibhar transposase. In some cases, the transposase utilized in a subject method is a Tn transposase, for example, a Tn transposase selected from Tn3, Tn5, Tn7, and Tn10. In some cases, the transposase is Tn5.

In some cases, the transposase is an engineered transposase. The engineered transposase can have different properties relative to the parent transposase from which it was derived. In some cases, the transposase is a hyperactive transposase. In some cases, the engineered transposase can be capable of binding nucleic acid molecules comprising modified nucleotides or nucleotide analogs. A transposase of the disclosure, for example, can be an engineered transposase which binds to any nucleic acid molecule sequence in an unbiased manner. In some cases, a transposase of the disclosure is an engineered or mutant transposase comprising a peptide fragment with at least about 50%, about 55%, about 60%, about 65%, about 70%, about 75%, about 80%, about 85%, about 90%, about 91%, about 92%, about 93%, about 94%, about 95%, about 96%, about 97%, about 98%, or about 99% amino acid sequence identity to a corresponding peptide fragment of the parent transposase. The peptide fragment can be at least about 10, about 15, about 20, about 25, about 30, about 35, about 40, about 45, about 50, about 60, about 70, about 80, about 90, about 100, about 150, about 200, about 250, about 300, about 400, or about 500 amino acids in length. An engineered or mutant transposase of the disclosure may have increased transposition activity compared to the parent transposase. In some cases, the engineered or mutant transposase has at least about 5%, 10%, 15%, 20%, 25%, 30%, 35%, 40%, 45%, or 50% greater activity than the parent transposase. An engineered or mutant transposase of the disclosure may have decreased transposition activity compared to the parent transposase. In some cases, an engineered or mutant transposase has at least about 5%, 10%, 15%, 20%, 25%, 30%, 35%, 40%, 45%, or 50% lower activity than the parent transposase.

In various embodiments of the aspects herein, transposon nucleic acids can be DNA, RNA or reverse transcribed RNA (e.g., cDNA). A transposon element of a transposon nucleic acid can be recognized by transposases and can be referred to as a recognition sequence. Recognition sequences can include the entire transposon nucleic acid or any portion thereof. In some cases, either both ends or a single end of the transposon nucleic acid can be recognized by a transposase. For example, Tn5 transposase can recognize a 19 bp sequence on either end of the transposon nucleic acid.

A transposon, also referred herein as a tag, a transposon nucleic acid, a transposon sequence, or a transposon segment, is a nucleic acid molecule that can be inserted into another nucleic acid molecule by a transposase. In some instances, the transposon is at least one of a single-stranded nucleic acid or a double-stranded nucleic acid. A transposon can be a double-stranded nucleic acid molecule, for example completely double-stranded or partially double-stranded, e.g., having a single-stranded overhang, having a bubble, having a loop, etc. A transposon often includes a transposon element or a transposase element. A transposon element or transposase element can refer to a nucleic acid molecule, or portion thereof, that includes nucleotide sequences that form a transposome with a transposase or integrase enzyme. In some instances, a transposon element is capable of forming a functional complex (e.g., transposome) with a transposase in a transposition reaction. Non-limiting examples of transposon elements include the 19-bp outer end ("OE") transposon end, inner end ("IE") transposon end, or "mosaic end" ("ME") transposon end recognized by, for example, a wild-type or mutant Tn5 transposase, or the R1 and R2 transposon end recognized by MuA transposases. In some cases, the transposon element or transposase element of a transposon used in embodiments herein is a ME transposon end. Transposon elements can comprise a nucleic acid or nucleic acid analogue suitable for forming a functional complex with the transposase or integrase. For example, the transposon element can comprise DNA, RNA, modified bases, non-natural bases, a modified backbone, or can comprise nicks in one or both strands.

In some cases, a transposon nucleic acid can join to a nucleic acid molecule in a sequence-dependent manner. For example, transposons *Passport, Himar1, Hsmar1, Frog Prince,* and *Sleeping Beauty* may preferentially join to nucleic acid molecule sequences enriched in 'TA'. Similarly, transposons *PiggyBac* and *PiggyBat* may preferentially join to the nucleic acid molecule sequences enriched in 'TTAA'. In some cases, a transposon nucleic acid can join to nucleic acid molecule sequences in a sequence-independent manner, or substantially sequence-independent manner. For example, transposons *Tol2* and *TcBuster* can join to any nucleic acid molecule sequences that are 8 bp long. Transposons can be substantially methylated, partially methylated, hemimethylated or substantially unmethylated.

In an aspect, the present disclosure provides a composition comprising a mixture of transposases comprising a first transposon nucleic acid (N1) and transposases comprising a second transposon nucleic acid (N2). In some cases, the relative ratio of N1 and N2 transposon nucleic acids in the composition is about 1:19, 2:18, 3:17, 4:16, 5:15, 6:14, 7:13, 8:12, 9:11, 10:10, 11:9, 12:8, 13:7, 14:6, 15:5, 16:4, 13:7, 12:8, or 19:1. In some cases, the relative ratio of N1 and N2 transposon nucleic acids in the composition is about 1:9, 2:8, 3:7, 4:6, 5:5, 6:4, 7:3, 8:2, or 9:1. In some cases, the relative ratio of N1 and N2 transposon nucleic acids in the composition is at least about 1:19, 2:18, 3:17, 4:16, 5:15, 6:14, 7:13, 8:12, 9:11, 10:10, 11:9, 12:8, 13:7, 14:6, 15:5, 16:4, 13:7, 12:8, or 19:1. In some cases, the relative ratio of N1 and N2 transposon nucleic acids in the composition is at least about 1:9, 2:8, 3:7, 4:6, 5:5, 6:4, 7:3, 8:2, or 9:1. In some embodiments, the molar concentration of N1 is less than that of N2. In some embodiments, the molar concentration of N2 is less than that of N1.

In various embodiments of disclosed herein, a double-stranded nucleic acid molecule generated from a nucleic acid molecules in the sample undergoes transposition or tagmentation after contact with the composition comprising a transposome. During transposition or tagmentation, one strand of a double-stranded transposon nucleic acid is often covalently linked to one strand of the double-stranded nucleic acid molecule (e.g., the "transferred strand" of the transposon nucleic acid). In some cases, the transferred strand is covalently linked to the 5' end of the one strand of the double-stranded nucleic acid molecule. The other strand of the transposon nucleic acid can be referred to as the "non-transferred strand." The non-transferred strand may not be linked to one strand of the double-stranded nucleic acid molecule. In cases where a target nucleic acid molecule for tagmentation is a double-stranded nucleic acid molecule, the top strand of the double-stranded nucleic acid molecule can be joined to a transposon nucleic acid via tagmentation by a first transposase while the bottom strand of the same double-stranded nucleic acid molecule can be joined at its 5' end to a another transposon nucleic acid via tagmentation by a another transposase. The joining of a top strand of a double-stranded nucleic acid molecule at its 5' end to a transposon nucleic acid and the bottom strand of a double-stranded nucleic acid molecule at its respective 5' end to a transposome sequence can yield an overhang fragment.

The transposition or tagmentation steps described herein use a transposome. In some cases, the transposome breaks the double-stranded nucleic acid molecule into fragments while covalently transferring the transposon nucleic acid to the first strand of the nucleic acid molecule fragment. In some cases, the transposase catalyzes insertion or transposition of the transposon nucleic acid to the target nucleic acid molecule by a cut and paste mechanism, and the target nucleic acid molecule is fragmented into shorter nucleic acid molecules (e.g., fragments). In some cases, the transposition reaction can include fragmentation prior to tagging of the nucleic acid molecule with the transposon nucleic acid. In some cases, fragmentation and tagging can occur simultaneously or substantially at the same time. In some cases, the transposase cleaves the nucleic acid molecule to produce a staggered cut that generates overhangs. The overhangs can be 1 base pair (bp), 2 bp, 3 bp, 4 bp, 5 bp, 6 bp, 7 bp, 8 bp, 9 bp, 10 bp, or longer in length. For example, Tn5 can cleave the nucleic acid molecule to produce 9 bp overhangs at 5' ends of the double stranded sequence. In some cases, the transposase cleaves the nucleic acid molecule to produce a blunt end cut.

In various embodiments of disclosed herein, the insertion of a transposon nucleic acid by a transposase can be at a random or substantially random site in the double-stranded nucleic acid molecule. In some instances, the transferred strand of a transposon nucleic acid can remain hybridized to the non-transferred strand following tagmentation. In some embodiments, a gap between the non-transferred strand and one strand of the double-stranded target nucleic acid molecule is formed. In some embodiments, the gap is filled by a polymerase and/or ligase. In some embodiments, the non-transferred strand of the transposon nucleic acid does not remain hybridized to the transferred strand. In some embodiments, the non-transferred strand of the transposon nucleic acid dissociates from the transferred strand, for example as a result of heat denaturation. In some embodiments, the non-transferred strand of the transposon nucleic acid is displaced from the transferred strand, for example by action of strand displacing polymerase. In some embodiments, the non-transferred strand is separated from the transferred strand prior to an extension reaction. In some embodiments, this gap is not filled in by a polymerase and/or ligase.

The double-stranded nucleic acid molecule described herein can be of any of a variety of lengths. In some instances, the double-stranded nucleic acid molecule has a length of at least about 1 kilobase (kb), 2 kb, 3 kb, 4 kb, 5 kb, 6 kb, 7 kb, 8 kb, 9 kb, 10 kb, 20 kb, 30 kb, 40 kb, 50 kb, 60 kb, 70 kb, 80 kb, 90 kb, 100 kb, 200 kb, 300 kb, 400 kb, 500 kb, 600 kb, 700 kb, 800 kb, 900 kb, 1 megabase (Mb), 2 Mb, 3 Mb, 4 Mb, 5 Mb, 6 Mb, 7 Mb, 8 Mb, 9 Mb, 10 Mb, 20 Mb, 30 Mb, 40 Mb, 50 Mb, 60 Mb, 70 Mb, 80 Mb, 90 Mb, 100 Mb, 200 Mb, 300 Mb, 400 Mb, 500 Mb, 600 Mb, 700 Mb, 800 Mb, or 900 Mb. In some instances, the double-stranded nucleic acid molecule has a length of about 1 kb, 2 kb, 3 kb, 4 kb, 5 kb, 6 kb, 7 kb, 8 kb, 9 kb, 10 kb, 20 kb, 30 kb, 40 kb, 50 kb, 60 kb, 70 kb, 80 kb, 90 kb, 100 kb, 200 kb, 300 kb, 400 kb, 500 kb, 600 kb, 700 kb, 800 kb, 900 kb, 1 Mb, 2 Mb, 3 Mb, 4 Mb, 5 Mb, 6 Mb, 7 Mb, 8 Mb, 9 Mb, 10 Mb, 20 Mb, 30 Mb, 40 Mb, 50 Mb, 60 Mb, 70 Mb, 80 Mb, 90 Mb, 100 Mb, 200 Mb, 300 Mb, 400 Mb, 500 Mb, 600 Mb, 700 Mb, 800 Mb, or 900 Mb. In some instances, the double-stranded nucleic acid molecule has a length of at most about 900 Mb, 800 Mb, 700 Mb, 600 Mb, 500 Mb, 400 Mb, 300 Mb, 200 Mb, 100 Mb, 90 Mb, 80 Mb, 70 Mb, 60 Mb, 50 Mb, 40 Mb, 30 Mb, 20 Mb, 10 Mb, 9 Mb, 8 Mb, 7 Mb, 6 Mb, 5 Mb, 4 Mb, 3 Mb, 2 Mb, 1 Mb, 900 kb, 800 kb, 700 kb, 600 kb, 500 kb, 400 kb, 300 kb, 200 kb, 100 kb, 90 kb, 80 kb, 70 kb, 60 kb, 50 kb, 40 kb, 30 kb, 20 kb, 10 kb, 9 kb, 8 kb, 7 kb, 6 kb, 5 kb, 4 kb, 3 kb, 2 kb, or 1 kb.

The size of the resulting overhang fragments described herein may be any of a variety of lengths. In some cases, the size of the resulting overhang fragments may be dependent on the length of the starting nucleic acid molecule. The size of the overhang fragments can depend on, in some cases, the concentration of transposase during tagmentation. In some cases, the overhang fragments comprises fragments having a length of at least 50 bases, 75 bases, 100 bases, 125 bases, 150 bases, 175 bases, 200 bases, 225 bases, 250 bases, 275 bases, 300 bases, 325 bases, 350 bases, 375 bases, 400 bases, 425 bases, 450 bases, 475 bases, 500 bases, 525 bases, 550 bases, 575 bases, 600 bases, 625 bases, 650 bases, 675 bases, 700 bases, 725 bases, 750 bases, 775 bases, 800 bases, 825 bases, 850 bases, 875 bases, 900 bases, 925 bases, 950 bases, 975 bases, 1 kb, 2 kb, 3 kb, 4 kb, 5 kb, 6 kb, 7 kb, 8 kb, 9 kb, 10 kb, 20 kb, 30 kb, 40 kb, 50 kb, 60 kb, 70 kb, 80 kb, 90 kb, or 100 kb. In some cases, the overhang fragments comprises fragments having a length of about 50 bases, 75 bases, 100 bases, 125 bases, 150 bases, 175 bases, 200 bases, 225 bases, 250 bases, 275 bases, 300 bases, 325 bases, 350 bases, 375 bases, 400 bases, 425 bases, 450 bases, 475 bases, 500 bases, 525 bases, 550 bases, 575 bases, 600 bases, 625 bases, 650 bases, 675 bases, 700 bases, 725 bases, 750 bases, 775 bases, 800 bases, 825 bases, 850 bases, 875 bases, 900 bases, 925 bases, 950 bases, 975 bases, 1 kb, 2 kb, 3 kb, 4 kb, 5 kb, 6 kb, 7 kb, 8 kb, 9 kb, 10 kb, 20 kb, 30 kb, 40 kb, 50 kb, 60 kb, 70 kb, 80 kb, 90 kb, or 100 kb. In some cases, the overhang fragments comprises fragments having a length of at most about 100 kb, 90 kb, 80 kb, 70 kb, 60 kb, 50 kb, 40 kb, 30 kb, 20 kb, 10 kb, 9 kb, 8 kb, 7 kb, 6 kb, 5 kb, 4 kb, 3 kb, 2 kb, 1 kb, 975 bases, 950 bases, 925 bases, 900 bases, 875 bases, 850 bases, 825 bases, 800 bases, 775 bases, 750 bases, 725 bases, 700 bases, 675 bases, 650 bases, 625 bases, 600 bases, 575 bases, 550 bases, 525 bases, 500 bases, 475 bases, 450 bases, 425 bases, 400 bases, 375 bases, 350 bases, 325 bases, 300 bases, 275 bases, 250 bases, 225 bases, 200 bases, 175 bases, 150 bases, 125 bases, 100 bases, 75 bases, or 50 bases.

The methods, compositions, reaction mixtures, kits, and systems described herein use a transposon nucleic acid for generating a dual end tagged nucleic acid molecules comprising a tag of the transposon nucleic acid or a portion thereof. In some instances, a transposon nucleic acid includes other nucleic acid molecules in addition to a transposon element. In some instances, the additional nucleic acid molecules are inserted into a target nucleic acid molecule via the transposition reaction. The additional nucleic acid molecules can include a primer binding site, such as a sequencing primer site and/or an amplification primer site. Additional nucleic acid molecules can also include a cleavage site, an anchor site, a reporter tag, a barcode, a cell barcode, a unique molecular identifier (UMI), or a mosaic end segment. A primer binding site can include sequences for sequencing primers to anneal to a nucleic acid in a sequencing reaction or other extension reactions. Such additional nucleic acid molecules can be useful in downstream nucleic acid molecule manipulation steps as well.

In various embodiments of disclosed herein, the transposase can be inactivated or removed before proceeding to the next step of a reaction following transposition. Inactivation or removal of the transposase can be useful in minimizing the inhibition to downstream reactions, such as extension reactions or amplification reactions that use tagged fragments as templates. Sometimes, the transposase is inactivated by a protease. In some instances, the protease used for inactivation is heat labile. In some instances, the protease is active at a temperature no more than 65°C. In some instances, the protease is inactive at a temperature greater than 65°C. In some instances, the protease is active at a temperature no more than 40°C, 45°C, 50°C, 55°C, 60°C, 65°C, 70°C, 75°C, or 80°C. In some instances, the protease is inactive at a temperature no less than 40°C, 45°C, 50°C, 55°C, 60°C, 65°C, 70°C, 75°C, or 80°C. In some instances, the protease is provided with the transposome and activated by a change in temperature and/or a buffer condition. In some instances, the protease is added after the transposome contacts the nucleic acid molecule. In some instances, the protease is added after the transposition. Alternatively or in combination, the transposase is removed by any of a variety of suitable methods, including purification or inactivation, for example via denaturation or enzymatic treatment. Sometimes, a chemical treatment is employed for removing the transposase. In some instances, the chemical treatment includes treating the tagged fragments with a detergent solution, such as an SDS solution. In some cases, the tagged fragments are not subjected to treatment to remove the transposase.

### Extension Reaction

In various embodiment of the disclosure herein, the dual-end tagged nucleic acid molecule may have 5' overhang fragments comprising a tag or a transposon nucleic acid. The overhang fragments can be subjected to an extension reaction to generate blunt-ended fragments. The extension reaction can be used to fill in an overhang to yield a blunt-end. In the extension reaction, one strand of an overhang fragment may function as a primer while the other strand functions as the template. The extension reaction may involve use of a polymerase. The polymerase may be a strand-displacing polymerase capable of displacing the non-transferred strand.

In some instances, the overhang fragments have a first transposon nucleic acid at a 5' end of a top strand and a first transposon nucleic acid at a 5' end of the bottom strand. After extension to fill the overhang(s), individual top strands and bottom strands may comprise the first transposon nucleic acid at a 5' end and a reverse complement of the first transposon nucleic acid at a 3' end. As a result of sequence complementarity, the 5' end of an individual top strand or bottom strand can base pair with its own 3' end, thereby forming a stem loop or hair pin structure.

In some instances, the overhang fragments have a first transposon nucleic acid at a 5' end of a top strand and a second transposon nucleic acid at a 5' end of the bottom strand. After extension to fill the overhang(s), individual top strands and bottom strands may comprise the first transposon nucleic acid at a 5' end and a reverse complement of the second transposon nucleic acid at a 3' end or the second transposon nucleic acid at a 5' end and a reverse complement of the first transposon nucleic acid at a 3' end.

A primer-directed extension reaction, or amplification, can be conducted to generate copies of the blunt-ended, dual-tagged nucleic acid molecule or blunt-ended fragments described herein. Primer extension can be conducted with a primer comprising the first transposon nucleic acid or a portion thereof. Alternatively or in combination, the primer extension can be conducted with a primer comprising the second transposon nucleic acid or a portion thereof. The primers can have a segment at a 5' end and a segment at a 3' end. In some cases, the primers can have additional segments interposed between the 5' and 3' ends or flanking the 5' and/or 3' ends. The segment at the 3' end can exhibit sequence complementarity to a blunt-ended fragment. The segment at the 5' end may also exhibit sequence complementarity to the blunt-ended fragment. The segment at the 3' end can exhibit sequence complementarity to the first or second transposon nucleic acid or a fragment thereof. The segment at the 5' end may also exhibit sequence complementarity to the first or second transposon nucleic acid or a fragment thereof. The segment at the 3' end may not exhibit sequence complementarity to the first or second transposon nucleic acid but rather a sequence of the nucleic acid molecule. The segment at the 5' end also may not exhibit sequence complementarity to the first or second transposon nucleic acid but rather a sequence of the nucleic acid molecule. The sequence of the nucleic acid molecule of the primer may be a segment of a gene of interest or its complementary segment. The segment at the 5' end may not exhibit sequence complementarity to any portion of a blunt-ended fragment and may comprise an additional sequence that is appended to the sequence of the blunt-ended fragment when the copies are generated.

In various embodiments described herein, the primer comprises the first transposon nucleic acid or a portion thereof. Use of a primer comprising the first transposon nucleic acid or a portion thereof may allow for the generation of copies of any blunt-ended fragment comprising the first transposon nucleic acid. In some cases, the primer comprises the second transposon nucleic acid or a portion thereof. Use of a primer comprising the second transposon nucleic acid or a portion thereof may allow for the generation of copies of any blunt-ended fragment comprising the second transposon nucleic acid. In some cases, copies of every blunt-ended fragment are generated. In some cases, copies of most blunt-ended fragments are generated. In some cases, copies of a majority of blunt-ended fragments are generated. In some embodiments, copies of at least 50%, 55%, 60%, 65%, 70%, 75%, 80%, 85%, 90%, or 95% of the blunt-ended fragments are generated during primer-directed extension. In some cases, this process can be referred to as single-primer amplification. In some cases, copies of the blunt-ended fragments are generated irrespective of the nucleic acid molecule sequence between the transposon nucleic acids. In some cases, use of a primer comprising the first transposon nucleic acid can allow for unbiased amplification of the blunt-ended fragments. In some cases, use of a primer comprising the second transposon nucleic acid can allow for unbiased amplification of the blunt-ended fragments.

In various embodiments described herein, the segment at the 3' end of a primer comprises gene-specific sequences. For example, where primer extension products comprising a particular gene sequence are desired for downstream analysis, the primer can comprise a segment at the 3' end capable of acting as a gene specific primer (e.g., having a gene-specific sequence). The gene-specific sequence of the primer can hybridize to a gene-specific tagged fragment and initiate primer extension. The extension reaction can select for, and in some cases, enrich a target sequence from a plurality of blunt-ended fragments. In some cases, primers having gene specific sequences corresponding to multiple genes can be used in combination to select for, and in some cases enrich, a plurality of gene specific blunt-ended fragments. For example, to generate extension products of tagged fragments corresponding to two gene sequences (e.g., gene 1 and gene 2), half of the primers may have a 3' segment with a sequence specific for gene 1 and half of the primers may have a 3' segment with a sequence specific for gene 2. For further example, to generate extension products of tagged fragments corresponding to three gene sequences (e.g., gene 1, gene 2, and gene 3), one-third of the primers may have a 3' segment with a sequence specific for gene 1, one-third of the primers may have a 3' segment with a sequence specific for gene 2, and one-third of the primers may have a 3' segment with a sequence specific for gene 3. The ratios of primers in a mixture can be optimized and/or adjusted as desired

In some cases, the gene specific sequence comprises a sequence targeting a cancer specific gene or a sequence implicated in cancer. In some cases, multiple primers, each being specific for one target gene, are utilized. In some cases, the primers of a subject method comprise a mixture of gene-specific primers and the mixture is used, for example, for multiplex processing. In some cases, the mixture of gene-specific primers target at least 5 genes (e.g., at least 10 genes, 15 genes, 20 genes, 25 genes, 50 genes, 100 genes, 200 genes, 300 genes, 400 genes, 500 genes, 600 genes, 700 genes, 800 genes, 900 genes, or 1,000 genes).

In various embodiments described herein, the segment at the 5' end of an extension primer may lack sequence complementarity to a tagged fragment. In some instances, the 5' end of an extension primer comprises sequences that may be utilized in downstream sample processing steps. For example, the segment at the 5' end of an extension primer comprises one or more amplification primer annealing sequences or complements thereof; one or more sequencing primer annealing sequences or complements thereof; one or more barcode sequences; one or more common sequences shared among multiple different primers; one or more restriction enzyme recognition sites; one or more probe binding sites or sequencing adapters (e.g., for attachment to a sequencing platform, such as a flow cell for massive parallel sequencing); one or more random or near-random sequences (e.g. one or more nucleotides selected at random from a set of two or more different nucleotides at one or more positions, with each of the different nucleotides selected at one or more positions represented in a pool of primers comprising the random sequence); and combinations thereof. The primer often comprises a sequence adapter. In some instances, the sequence adapter comprises at least one of sequencing primer binding sequences (e.g., Read1 or Read2), unique molecular identifiers or barcode sequences (e.g., i5, i7) and/or flow cell binding sequences (e.g., P5, P7). In some cases, the adapters are attached or appended to the 5' and/or 3' ends of the primers, for example by ligation, amplification, or a combination thereof.

A first plurality of blunt-ended fragments or fragments thereof can be contacted with a second set of transposome complexes to generate a second plurality of overhang fragments that can comprise a second transposon nucleic acid at a 5' end. Individual transposome complexes of the second set can comprise the second transposon nucleic acid. In some embodiments, the second set of transposome complexes is a mixture of homodimer transposome complexes and further comprises transposome complexes having the first transposon nucleic acid. In some embodiments, the mixture further comprises transposome complexes having a third transposon nucleic acid, a fourth transposon nucleic acid, a fifth transposon nucleic acid, etc, as desired. In some embodiments, the second set of transposome complexes comprises heterodimer transposome complexes, individual transposome complexes comprising the first transposon nucleic acid and the second transposon nucleic acid. By using a mixture of homodimer transposome complexes having different transposon nucleic acids or heterodimer transposome complexes having at least two transposon nucleic acids, individual overhang fragments of the resulting second plurality of overhang fragments can have either a first transposon nucleic acid, second transposon nucleic acid, third transposon nucleic acid, fourth transposon nucleic acid, etc, at the 5' end. The second plurality of overhang fragments can comprise a plurality of fragments having different transposon ends. In some embodiments, the second set of transposome complexes comprises homodimer transposome complexes having a transposon nucleic acid different from the first transposon nucleic acid.

The size of the overhang fragments can be of any of a variety of lengths. In some instances, the size of the overhang fragments may be dependent on the length of the blunt-ended fragments. The size of the overhang fragments can depend on, in some cases, the concentration of transposase during tagmentation. In some instances, the overhang fragments comprises fragments having a length of at least 10 bases, 20 bases, 30 bases, 40 bases, 50 bases, 75 bases, 100 bases, 125 bases, 150 bases, 175 bases, 200 bases, 225 bases, 250 bases, 275 bases, 300 bases, 325 bases, 350 bases, 375 bases, 400 bases, 425 bases, 450 bases, 475 bases, 500 bases, 525 bases, 550 bases, 575 bases, 600 bases, 625 bases, 650 bases, 675 bases, 700 bases, 725 bases, 750 bases, 775 bases, 800 bases, 825 bases, 850 bases, 875 bases, 900 bases, 925 bases, 950 bases, 975 bases, or 1 kb. In some embodiments, the overhang fragments comprises fragments having a length of at most 1 kb, 975 bases, 950 bases, 925 bases, 900 bases, 875 bases, 850 bases, 825 bases, 800 bases, 775 bases, 750 bases, 725 bases, 700 bases, 675 bases, 650 bases, 625 bases, 600 bases, 575 bases, 550 bases, 525 bases, 500 bases, 475 bases, 450 bases, 425 bases, 400 bases, 375 bases, 350 bases, 325 bases, 300 bases, 275 bases, 250 bases, 225 bases, 200 bases, 175 bases, 150 bases, 125 bases, 100 bases, 75 bases, 50 bases, 40 bases, 30 bases, 20 bases, or 10 bases.

The overhang fragments can be subjected to an additional extension reaction to generate a second plurality of blunt-ended fragments. The additional extension reaction can be useful in filling in an overhang to yield a blunt-end. In the additional extension reaction, one strand of an overhang fragment may function as a primer while the other strand functions as a template. The extension reaction may involve use of a polymerase. The polymerase may be a strand-displacing polymerase capable of displacing a non-transferred strand.

The second blunt-ended fragments or derivatives thereof can be sequenced to generate sequence reads, thereby characterizing the double-stranded nucleic acid molecule in the sample. In some instances, the second blunt-ended fragments are subject to further nucleic acid processing in preparation for sequencing analysis. In some instances, sequencing primer binding sequences (e.g., Read1 or Read2), unique molecular identifiers or barcode sequences (e.g., i5, i7) and/or flow cell binding sequences (e.g., P5, P7) are attached or appended to the 5' and/or 3' ends of the fragments, for example by ligation, amplification, or a combination thereof.

Some embodiments of the present disclosure comprise primer extension and amplification reactions, such as generating extension products and amplifying extension products. Primer extension reactions can involve changes in temperature (thermocycling) or a constant temperature (isothermal). In some embodiments, primer extension reactions comprise polymerase chain reaction (PCR). PCR involves cycling through multiple stages of denaturation, annealing of primer pairs to opposite strands, and primer extension to exponentially increase copy numbers of the target sequence, at least some of these stages generally occurring at different reaction temperatures. Non-limiting examples of PCR amplification techniques are quantitative PCR (qPCR or realtime PCR), reverse transcription PCR (RT-PCR), digital PCR (dPCR or dePCR), target-specific PCR, and quantitative reverse transcription PCR (qRT- PCR), dual primer PCR (dpPCR), single primer PCR (spPCR). Examples of polymerase enzymes that can be used for PCR are thermostable polymerases, including but not limited to, Thermus thermophilus HB8; mutant Thermus oshimai; Thermus scotoductus; Thermus thermophilus 1B21; Thermus thermophilus GK24; Thermus aquaticus polymerase (AmpliTaq^{®} FS or Taq (G46D; F667Y), Taq (G46D; F667Y; E6811), and Taq (G46D; F667Y; T664N; R660G); Pyrococcus furiosus polymerase; Thermococcus gorgonarius polymerase; Pyrococcus species GB-D polymerase; Thermococcus sp. (strain 9° N-7) polymerase; Bacillus stearothermophilus polymerase; Tsp polymerase; ThermalAce^{™} polymerase (Invitrogen); Thermus flavus polymerase; Thermus litoralis polymerase; Thermus Z05 polymerase; delta Z05 polymerase (e.g. delta Z05 Gold DNA polymerase); and mutants, variants, or derivatives thereof. Additional examples of polymerase enzymes that can be used for PCR are non-thermostable polymerases, including, but are not limited to DNA polymerase I; mutant DNA polymerase I, including, but not limited to, Klenow fragment and Klenow fragment (3' to 5' exonuclease minus); T4 DNA polymerase; mutant T4 DNA polymerase; T7 DNA polymerase; mutant T7 DNA polymerase; phi29 DNA polymerase; and mutant phi29 DNA polymerase.

In some embodiments, primer extension and amplification reactions comprise isothermal reactions. Non-limiting examples of isothermal amplification technologies are ligase chain reaction (LCR); transcription mediated amplification (TMA); nucleic acid sequence-based amplification (NASBA); signal mediated amplification of RNA technology (SMART); strand displacement amplification (SDA); thermophilic SDA; rolling circle amplification (RCA); loop-mediated isothermal amplification of DNA (LAMP); helicase-dependent amplification (HDA) ; single primer isothermal amplification (SPIA); and circular helicase-dependent amplification (cHDA).

In various embodiments of the aspects herein, a hot-start polymerase is used for extension and/or amplification. The term "hot-start" generally refers to a means of limiting the availability of an essential reaction component (e.g., a polymerase) when the reaction mixture is maintained at a first temperature (typically a lower temperature) until a second temperature (typically a higher temperature) is reached which allows the essential component to participate in the reaction. Hot-start reactions typically involve incubation at a first (e.g., lower) temperature and subsequent elevation to a second (e.g., higher) temperature which allows the desired reaction to take place. Activation of the hot start reaction can be achieved by incubating a reaction mixture at a temperature which is equal to or higher than the primer hybridization (annealing) temperature. Use of a temperature which is equal to or greater than the primer hybridization temperature can ensure primer binding specificity. The length of incubation required to recover enzyme activity depends on the temperature and pH of the reaction mixture and on the stability of the enzyme. A wide range of incubation conditions are usable; optimal conditions may be determined empirically for each reaction. The solutions can be optionally heated to and held at a first temperature for a first period of time suitable for hot-start activation of the nucleic acid polymerases.

Non-limiting exemplary hot start mechanisms include, but are not limited to, antibodies or combinations of antibodies that block nucleic acid polymerase activity at lower temperatures and which dissociate from the polymerase at elevated temperatures; affibodies or combinations of affibodies, sometimes referred to as antibody mimetics; oligonucleotides that block nucleic acid polymerase activity at lower temperatures and which dissociate from the polymerase at elevated temperatures; reversible chemical modification of the nucleic acid polymerase such that the nucleic acid polymerase activity is blocked at lower temperatures and the modifications reverse or dissociate at elevated temperatures; amino acid mutations of the nucleic acid polymerase that provide reduced activity at lower temperatures; nucleic acid polymerase fusion proteins including hyperstable DNA binding domains and topoisomerases; ligands that inhibit the nucleic acid polymerase in a temperature-dependent manner; single-stranded binding proteins that sequester primers at low temperatures; thermostable pyrophosphatase which hydrolyzes inorganic pyrophosphate at elevated temperatures; thermolabile blockers, such as a polymerase blocking protein; primer competitor sequences; modified primer constructs ; modified primers that improve hybridization selectivity; primers with 3' modifications that are removable by 3'-5' exonuclease activity; primers with modified nucleobases that are removable by UV irradiation; primer modifications that are removable by thermal deprotection; or modification of the dNTPs with thermolabile modification groups. Agents that are used as hot start mechanisms, such as, but not limited to, antibodies, oligonucleotides, Affibodies, chemical modifications, etc., may be referred to as "hot start inhibitors."

In some embodiments, a hot start composition comprises an antibody specific for the polymerase. In some embodiments, a hot start composition comprises an antibody specific for the polymerase, which is bound to the polymerase. In some embodiments, a hot start composition comprises an inhibitor specific for the polymerase, which is bound to the polymerase. In some embodiments, the inhibitor comprises an Affibody. In some embodiments, the inhibitor comprises an oligonucleotide. In some embodiments, the inhibitor comprises a chemical modification. A number of hot start polymerases are available from various commercial sources, such as Applied Biosystems; Bio-Rad; eEnzyme LLC; Eppendorf North America; Finnzymes Oy; GeneChoice, Inc.; Invitrogen; Jena Bioscience GmbH; MTDSCI; Minerva Biolabs GmbH; New England Biolabs; Novagen; Promega; QIAGEN; Roche Applied Science; Sigma- Aldrich; Stratagene; Takara Minis Bio; USB Corp.; Yorkshire Bioscience Ltd; and the like.

In some embodiments of any of the various aspects of the present disclosure, a primer may comprise one or more portions. For example, a primer may comprise one or more amplification primer annealing sequences or complements thereof; one or more sequencing primer annealing sequences or complements thereof; one or more barcode sequences; one or more common sequences shared among multiple different primers; one or more restriction enzyme recognition sites; one or more probe binding sites or sequencing adapters (e.g., for attachment to a sequencing platform, such as a flow cell for massive parallel sequencing); one or more random or near-random sequences (e.g. one or more nucleotides selected at random from a set of two or more different nucleotides at one or more positions, with each of the different nucleotides selected at one or more positions represented in a pool of primers comprising the random sequence); and combinations thereof.

Non-limiting examples of next-generation sequencing methods are single-molecule real-time sequencing, ion semiconductor sequencing, pyrosequencing, sequencing by synthesis, sequencing by ligation, and chain termination. Sequencing adapters for flow cell attachment may comprise any suitable sequence compatible with next generation sequencing systems, e.g., 454 Sequencing, Ion Torrent Proton or PGM, and Illumina X10. Non-limiting examples of sequencing adapters for next generation sequencing methods include P5 and P7 adapters suitable for use with Illumina sequencing systems; TruSeq Universal Adapter; and TruSeq Indexed Adapter. In some embodiments, a sequencing adapter can be used to enrich, e.g., via amplification, such as polymerase chain reaction (PCR), for nucleic acid molecules comprising the adapter sequence. Sequencing adapters can further comprise a barcode sequence and/or a sample index sequence.

In some embodiments, a primer comprises a barcode sequence. A barcode sequence refers to a known nucleic acid sequence that allows some feature of a nucleic acid molecule with which the barcode is associated to be identified. Barcodes can each have a length within a range of 5 to 35 nucleotides, 6 to 30 nucleotides, or 8 to 20 nucleotides. In some embodiments, barcodes are at least 3, 4, 5, 6, 7, 8, 9, 10, 11, 12, 13, 14, or 15 nucleotides in length. In some embodiments, barcodes are less than 6 nucleotides in length. In some embodiments, barcodes associated with some target nucleic acid molecules may be a different length than barcodes associated with other target nucleic acid molecules. The melting temperatures of barcodes within a set can be within ±10 °C of one another, within ±5 °C of one another, or within ±2 °C of one another. Barcodes can be members of a minimally cross-hybridizing set. For example, the nucleotide sequence of each member of such a set can be sufficiently different from that of every other member of the set that no member can form a stable duplex with the complement of any other member under moderate or stringent hybridization conditions. The nucleotide sequence of each member of a minimally cross-hybridizing set can differ from those of every other member by at least two nucleotides. Some barcode technologies are described in Winzeler et al. (1999) Science 285:901; Brenner (2000) Genome Biol. 1 : 1 Kumar et al. (2001) Nature Rev. 2:302; Giaever et al. (2004) Proc. Natl. Acad. Sci. USA 101 :793; Eason et al. (2004) Proc. Natl. Acad. Sci. USA 101: 11046; and Brenner (2004) Genome Biol. 5:240.

### Amplification Products

Amplification products (also referred to as amplicons) produced according to methods herein can be analyzed by sequencing. A variety of sequencing methodologies are available for sequencing amplification products. In some embodiments, high-throughput sequencing methodologies are used. Non-limiting examples of sequencing methodologies that can be used include sequencing systems manufactured by Illumina (sequencing systems such as HiSeq^{®} and MiSeq^{®}), Life Technologies (Ion Torrent^{®}, SOLiD^{®}, etc.), Roche's 454 Life Sciences systems, Pacific Biosciences systems, etc. In some embodiments, sequencing comprises use of HiSeq^{®} and MiSeq^{®} systems to produce reads of about or more than about 50, 75, 100, 125, 150, 175, 200, 250, 300 nucleotides or more in length. In some embodiments, sequencing comprises a sequencing-by-synthesis process, where individual nucleotides are identified iteratively, as they are added to the growing primer extension product. Pyrosequencing is an example of a sequence by synthesis process that identifies the incorporation of a nucleotide by assaying the resulting synthesis mixture for the presence of by-products of the sequencing reaction, namely pyrophosphate. In particular, a primer/template/polymerase complex is contacted with a single type of nucleotide. If that nucleotide is incorporated, the polymerization reaction cleaves the nucleoside triphosphate between the α and β phosphates of the triphosphate chain, releasing pyrophosphate. The presence of released pyrophosphate is then identified using a chemiluminescent enzyme reporter system that converts the pyrophosphate, with AMP, into ATP, and then measures ATP using a luciferase enzyme to produce measurable light signals. Where light is detected, the base is incorporated, where no light is detected, the base is not incorporated. Following appropriate washing steps, the various bases are cyclically contacted with the complex to sequentially identify subsequent bases in the template sequence. See, e.g., U.S. Pat. No. 6,210,891.

In various embodiments of the present disclosure, amplification products are purified prior to sequencing. Amplification products can be purified by various methods. Amplification products may be purified to remove excess or unwanted reagents, reactants, or products. Amplification products may further be purified by size, sequence, or other physical or chemical characteristic. In some embodiments, amplicons may be subjected to size exclusion chromatography. In some embodiments, amplification products may be subjected to fragment excision from gels and gel filtration (e.g. to enrich for fragments larger than about 300, 400, 500, or more nucleotides in length); as well as SPRI beads (Agencourt AMPure XP) for size selection by fine-tuning the binding buffer concentration. For example, the use of 0.6x binding buffer during mixing with DNA fragments may be used to preferentially bind DNA fragments larger than about 500 base pairs (bp).

### Reaction Times

In various methods, compositions, reaction mixtures, and kits provided herein, the reverse transcription and tagmentation may be performed in a single step in a single reaction chamber and provide a faster way to generate a cDNA library. As described herein, the cDNA library may be generated by reverse-transcribing an RNA sample in a sample; introducing complementary cleavage sites into the DNA/RNA hybrid molecule; attaching at cleavage site 5' ends a population of 5' overhang tags; and synthesizing complementary segments to the 5' overhang tags from 3' recessed ends of the DNA/RNA hybrid molecule to form blunt ended DNA/RNA hybrid molecules. In some instances, the cDNA library is generated in no more than 5 hours, 4 ½ hours, 4 hours, 3 ½ hours, 3 hours, 2 ½ hours, 2 hours, 1 ½ hours, 1 hour, 55 minutes, 50 minutes, 45 minutes, 40 minutes, 35 minutes, 30 minutes, 25 minutes, 20 minutes, 15 minutes, 10 minutes, or 5 minutes. In some instances, the cDNA library is generated in at least 5 hours, 4 ½ hours, 4 hours, 3 ½ hours, 3 hours, 2 ½ hours, 2 hours, 1 ½ hours, 1 hour, 55 minutes, 50 minutes, 45 minutes, 40 minutes, 35 minutes, 30 minutes, 25 minutes, 20 minutes, 15 minutes, 10 minutes, or 5 minutes. In some instances, the cDNA library is generated in no more than 2 hours. In some instances, the cDNA library is generated in no more than 45 minutes.

The various embodiments described herein may provide a faster way to generate a sequencing library by performing the reverse transcription and tagmentation in a single step in a single reaction chamber. Often, the sequencing library is generated after the index PCR step. In some instances, the sequencing library is generated in no more than 10 hours, 9 ½ hours, 9 hours, 8 ½ hours, 8 hours, 7 ½ hours, 7 hours, 6 ½ hours, 6 hours, 5 ½ hours, 5 hours, 4 ½ hours, 4 hours, 3 ½ hours, 3 hours, 2 ½ hours, 2 hours, 1 ½ hours, 1 hour, 55 minutes, 50 minutes, 45 minutes, 40 minutes, 35 minutes, or 30 minutes. In some instances, the sequencing library is generated in at least 5 hours, 4 ½ hours, 4 hours, 3 ½ hours, 3 hours, 2 ½ hours, 2 hours, 1 ½ hours, 1 hour, 55 minutes, 50 minutes, 45 minutes, 40 minutes, 35 minutes, 30 minutes, 25 minutes, 20 minutes, or 15 minutes. In some instances, the sequencing library is generated in no more than 2 hours. In some instances, the sequencing library is generated in no more than 30 minutes of time additional to the cDNA library generation time hour. In some instances, the sequencing library is generated in no more than 15 minutes of time additional to the cDNA library generation time hour.

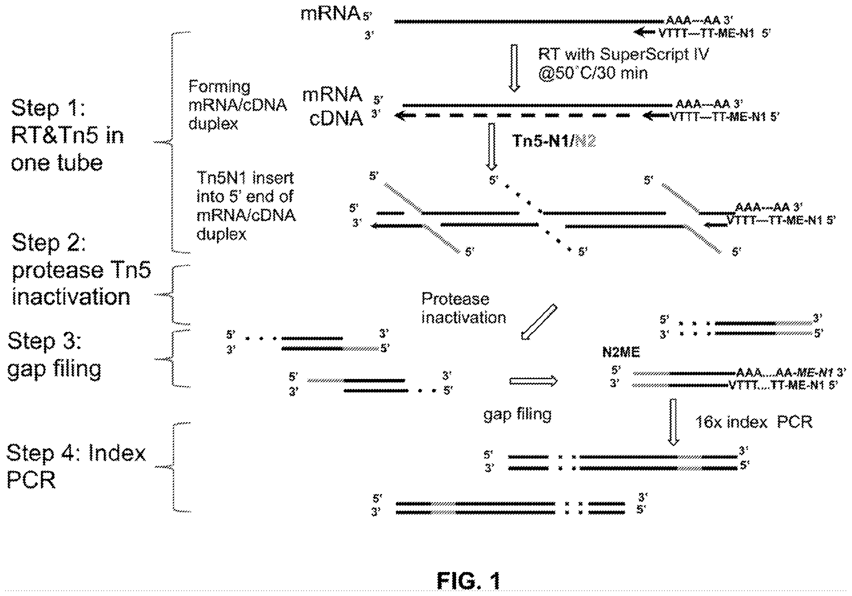

An example of the disclosure provided herein is illustrated in FIG. 1. As shown in FIG. **1****,** a bulk total mRNA molecule from a sample undergoes reverse transcription and tagmentation reaction in a single reaction chamber. The mRNA molecule is contacted with a reverse transcriptase and a reverse transcriptase primer comprising a first tag (denoted as N1), a mosaic end (denoted as ME), and a poly T segment. Often, the first tag is a first transposon segment. The reverse transcriptase primer as illustrated comprises the N1 at its 5' end, followed by ME, and poly T segment toward its 3' end. In some instances, the reverse transcriptase primer may be N1ME-T20VN. The reverse transcriptase primer hybridizes to the poly A tail at the 3' end of the mRNA molecule, and the reverse transcriptase synthesizes a cDNA molecule complementary to the mRNA molecule, extending from the reverse transcriptase primer in a '5 to 3' direction. The synthesis of cDNA molecule is illustrated as dashed lines in **FIG. 1**. In some instances, the reverse transcriptase is SuperScript IV reverse transcriptase, and the reverse transcription reaction takes place at 50°C for 30 minutes. The mRNA-cDNA hybrid is contacted with a transposome to undergo a transposition or tagmentation reaction. The transposome comprises a transposase and at least a first tag or a second tag, wherein the second tag (denoted as N2) comprises a second transposon segment. Sometimes, the transposome comprises a Tn5 and a first tag (N1) or a second tag (N2) or both. In some instances, the first or the second tag may further comprise an ME segment linked to the tag. In the tagmentation reaction, the transposome inserts the N1 or N2 tag at the cleaved 5' end of the mRNA-cDNA hybrid to generate a plurality of dual-end tagged mRNA-cDNA hybrid fragments having overhang tags of N1, N2, or both. The tags are illustrated as gray lines and dotted lines in **FIG. 1**, where dotted line represents the first tag comprising N1 and represents a continuous molecule without a break and gray line represent the second tag comprising N2. The dual-end tagged mRNA-cDNA hybrid fragments comprises a double-stranded mRNA-cDNA fragment of the double-stranded mRNA-cDNA hybrid having a 5' overhang tag extending from the 5' end of mRNA strand and a 5' overhang tag extending from the 5' end of cDNA strand, where the 5' overhang tag comprises at least one of N1 or N2. In some cases, the 5' overhang tags on one fragment are different tags. In some cases, the 5' overhang tags further comprises an ME segment. After the reverse transcription and tagmentation reaction, the transposase is inactivated. In some instances, a protease is introduced to the reaction chamber to inactivate the transposase. In some instances, the protease is active at a temperature of no more than 65°C and can be inactivated by heating to a temperature of at least 65°C after the transposases have been inactivated. Then, the plurality of dual-end tagged mRNA-cDNA hybrid fragments having overhang tags are subject to a gap filling or an extension reaction to generate a plurality of blunt-ended, dual-end tagged mRNA-cDNA hybrid fragments. In the extension reaction, complementary segments to the 5' overhang tags from 3' recessed ends of the mRNA-cDNA hybrid fragments are synthesized to form blunt-ended mRNA-cDNA hybrid fragments. In some cases, the extension reaction utilizes a DNA polymerase and deoxyribonucleotides to synthesize the complementary segments to the overhang tags. The blunt ended, dual-end tagged mRNA-cDNA hybrid fragments comprises two individual strands, where an individual strand comprises a tag at a 5' end of the fragment, an mRNA or cDNA fragment, and a reverse complement of another tag at a 3' end of the fragment. In some instances, the individual strand comprises a first tag and a ME segment (N1ME) a 5' end, mRNA or cDNA fragment, and a reverse complement of a second tag and a ME segment (N2'ME) at a 3' end, which is represented by a series of a dotted line, a black line, and a gray line, respectively, in **FIG. 1**. In some instances, the individual strand comprises a second tag and a ME segment (N2ME) a 5' end, mRNA or cDNA fragment, and a reverse complement of a first tag and a ME segment (N1'ME) at a 3' end, which is represented by a series of a gray line, a black line, and a dotted line, respectively, in **FIG. 1**. Next, a primer-directed extension reaction, or an amplification reaction, is performed to generate copies of a plurality of blunt-ended cDNA fragments. Use of primers comprising the first tag or a portion thereof or primers comprising the second tag or a portion thereof may allow for the generation of copies of any blunt-ended fragment comprising the first tag or the second tag, respectively. In some cases, the primers comprise sequence adapters, which are shown as black lines in **FIG. 1**. In some cases, the primers are P5-I5-N1, P7-i7, N2, P5, and P7. In some cases, the primer-directed extension reaction is index PCR. In some cases, 16 cycles of index PCR are performed. In some cases, at least 2, 3, 4, 5, 6, 7, 8, 9, 10, 11, 12, 13, 14, 15, 16, 17, 18, 19, 20 cycles of index PCR are performed. After the amplification, the plurality of blunt-ended cDNA fragments comprise individual strands comprising a tag at a 5' end of a cDNA fragment, the cDNA fragment, a reverse complement of another tag at a 3' end of the cDNA fragment, and a sequence adapter at either 5' or 3' end. In some cases, the individual strand comprises a first tag (N1) at a 5' end of a cDNA fragment, the cDNA fragment, and a reverse complement of a second tag at a 3' end (N2') of the cDNA fragment, and a sequence adapter at 3' end of N2', which is represented by a series of a dotted line, a black line, a gray line, and a black line, respectively, in **FIG. 1**. In some cases, the individual strand comprises a sequence adapter at 5' end of a second tag, a second tag (N2) at a 5' end of a cDNA fragment, the cDNA fragment, and a reverse complement of a first tag at a 3' end (N1') of the cDNA fragment, which is represented by a series of a black line, a gray line, a black line, and a dotted line, respectively, in **FIG. 1**.

The various embodiments described herein provide various capabilities and advantages in generating a cDNA library. The disclosure provided herein offers the capability to obtain an mRNA or a specific transcript using a gene specific oligonucleotide as the reverse transcription primer in a single step. The disclosure provided herein allows for a shorter extension time during preamplification that enables a more streamlined workflow and faster turnaround time. In some cases, the extension time during preamplification is improved to 3 minutes as compared to 6 minutes by the SMART-seq method. In some cases, the extension time during preamplification is improved to 1 minute, 2 minutes, 3 minutes, 4 minutes, or 5 minutes. In some cases, the extension time during preamplification is improved by 1 minute, 2 minutes, 3 minutes, 4 minutes, 5 minutes, or 6 minutes. In some cases, the extension time during preamplification is improved by at least 1 minute, 2 minutes, 3 minutes, 4 minutes, 5 minutes, or 6 minutes. The disclosure provided herein provides for almost full length coverage of mRNA. In some cases, the extent of the mRNA coverage depends on extend of reverse transcriptase, time, Tn5, and expression level of the mRNA.

The embodiments described herein provide various advantages in generating a cDNA or other library. In some embodiments, the tagmentation and the reverse transcription is performed in a single step. In some cases, the Tn5 insertion and reverse transcription is performed in a single step. The tagmentation uses a DNA-RNA hybrid as a template. In some cases, the tagmentation comprises Tn5 insertion, which uses a DNA-RNA hybrid as a template. The disclosure provided herein does not need a TSO oligonucleotide or second strand synthesis.

In **FIGS. 1-5**, the dashed lines and dotted lines represent continuous molecules and not a break in the molecule. Continuous molecules are depicted by dotted lines, dashed lines, black lines, and gray lines in the figures provided herein. The gray lines and dotted lines in the figures herein represent transposome-enabled tags of the nucleic acid molecules, which are continuous molecules.

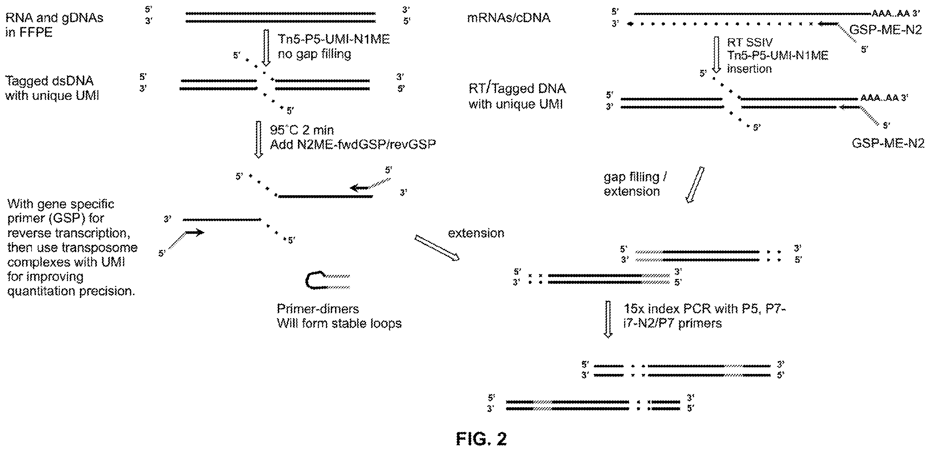

Transposome enabled DNA-RNA sequencing from genomic DNA and mRNA molecules is illustrated in in **FIG. 2**. This example provides DNA-RNA sequencing with universal digital counting to generate multiplex gene panels from both genomic DNA and mRNA from a single FFPE sample. The use of a gene specific primer for reverse transcription and of transposomes with UMI improves the quantitation precision. As shown in **FIG. 2**, the genomic DNA molecules from the FFPE sample undergo tagmentation without gap filling. In some cases, the tagmentation utilizes transposomes comprising a transposase and a nucleic acid molecule comprising a first tag (N1) linked to a ME segment. In some cases, the tagmentation utilizes transposomes comprising Tn5 and a nucleic acid molecule comprising P5-UMI-N1ME (Tn5-P5-UMI-N1ME). The tags are illustrated as gray lines and dotted lines in **FIG. 2**, where dotted line represents the first tag comprising N1 and and gray line represent the second tag comprising N2. In **FIG. 2** and throughout, both continuous grey lines and black dotted lines represent continuous nucleic acid strands, and white space between dots is not intended to represent breakpoints in nucleic acids. The tagmentation of the genomic DNA generates a plurality of tagged double-stranded DNA fragments, where the individual tagged double-stranded DNA fragment has a 5' end overhang fragment comprising a UMI or a tag that identifies a reaction batch and an N1 tag. The tagged double-stranded DNA fragments are subjected to 95°C for 2 minutes and gene specific primers are added to the reaction mixture for a primer-directed extension reaction. In some cases, the primers comprise a second tag (N2) linked to a ME segment and a gene specific segment in the forward or reverse direction. In some cases, the primers comprise N2ME and a gene specific segment in the forward or reverse direction. In some cases, the primers comprising a gene specific segment in the forward or reverse direction is referred to as a gene specific primer (GSP). The resulting dual end tagged, double stranded DNA segments comprise a 5' overhang fragment of a first tag and a 5' overhang fragment of a second tag. Next, a primer-directed extension reaction is performed to generate copies of the blunt-ended, dual end tagged DNA fragments. In some cases, the primers comprise sequence adapters, which are shown black lines at the end of the nucleic acid molecule in **FIG. 2**. In some cases, the primers are P5, P7-i7-N2, and P7. In some cases, the primer dimers can form stable loops. In some cases, the primer-directed extension reaction is index PCR. In some cases, 15 cycles of index PCR are performed. In some cases, at least 2, 3, 4, 5, 6, 7, 8, 9, 10, 11, 12, 13, 14, 15, 16, 17, 18, 19, 20 cycles of index PCR are performed.

The mRNA molecules from the same FFPE sample are subjected to reverse transcription and tagmentation in a single reaction chamber. The mRNA molecule is contacted with a reverse transcriptase and a reverse transcriptase primer comprising the second tag (N2), a mosaic end (ME), and a gene specific segment. The reverse transcriptase primer hybridizes to the mRNA molecule having a complement to the gene specific segment, and the reverse transcriptase synthesizes a cDNA molecule complementary to the mRNA molecule, extending from the reverse transcriptase primer. In some instances, the reverse transcriptase is SuperScript IV reverse transcriptase, and the reverse transcription reaction takes place at 50°C for 30 minutes, although as indicated above, alternatives are contemplated. The mRNA-cDNA hybrid undergoes the tagmentation reaction. The transposome comprises a transposase and the first tag (N1). Sometimes, the transposome comprises a Tn5 and a nucleic acid molecule comprising a first tag (N1), a ME segment, a P5 segment, and a UMI segment. In some cases, the transposome comprises Tn5-P5-UMI-N1ME. The tagmentation reaction generates a plurality of dual end tagged double-stranded mRNA-cDNA fragments, where the individual dual tagged mRNA-cDNA fragment has a 5' end overhang tag comprising a UMI or a tag that identifies a reaction batch and an N1 tag at the cleaved 5' end. Then, the plurality of dual-end tagged mRNA-cDNA fragments having 5' overhang tags are subject to a gap filling or an extension reaction to generate a plurality of blunt-ended, dual-end tagged mRNA-cDNA fragments. Next, a primer-directed extension reaction is performed to generate copies of the blunt-ended, dual end tagged DNA fragments from the blunt-ended, dual-end tagged mRNA-cDNA fragments. In some cases, the primers comprise sequence adapters. In some cases, the primers are P5, P7-i7-N2, and P7. In some cases, the primer-directed extension reaction is index PCR. In some cases, 15 cycles of index PCR are performed. In some cases, at least 2, 3, 4, 5, 6, 7, 8, 9, 10, 11, 12, 13, 14, 15, 16, 17, 18, 19, 20 cycles of index PCR are performed.

After the amplification, the plurality of blunt-ended DNA fragments from the genomic DNA and mRNA molecules comprise individual strands comprising a tag at a 5' end of a DNA fragment, the DNA fragment, a reverse complement of another tag at a 3' end of the DNA fragment, and a sequence adapter at either 5' or 3' end. In some cases, the individual strand comprises a first tag (N1) at a 5' end of a cDNA fragment, the DNA fragment, a gene specific segment or its complement, and a reverse complement of a second tag at a 3' end (N2') of the cDNA fragment. In some cases, the individual strand comprises a second tag (N2) at a 5' end of a cDNA fragment, a gene specific segment or its complement, the cDNA fragment, and a reverse complement of a first tag at a 3' end (N1') of the cDNA fragment.

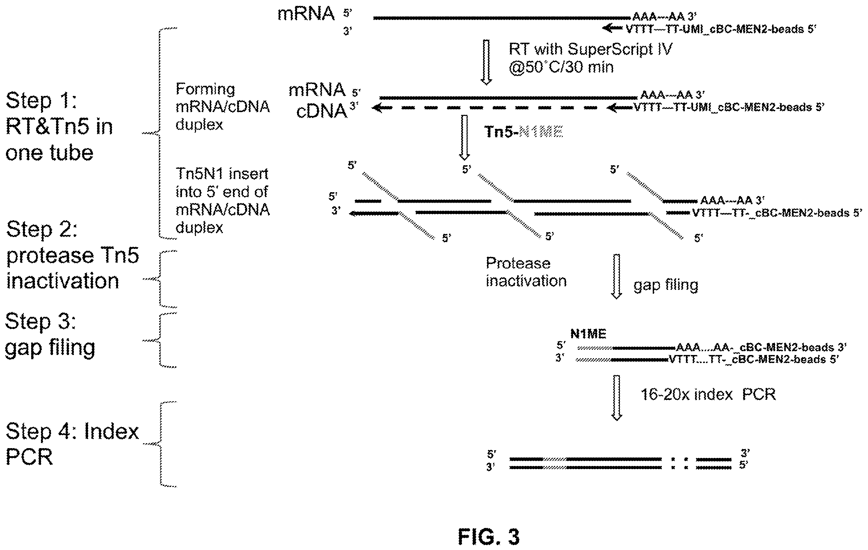

**FIG. 3** illustrates the capability to capture only the 3' end reads of an mRNA molecule for a cDNA library by the disclosure provided herein. This provides for a capability for digital counting of 10k single cell mRNAs profiling. In some embodiments, 10k single cell mRNAs profiling can be obtained by amplifying the 3' ends of mRNAs. The mRNA molecules from a single cell are subjected to reverse transcription and tagmentation in a single chamber in a single step. The mRNA molecules from a single cell are contacted with a reverse transcriptase and a reverse transcriptase primer comprising a second tag (denoted as N2), a mosaic end (denoted as ME), a cell bar code (cBC), a UMI segment, and a poly T segment, where the reverse transcriptase primer is bound at a 5' end to a bead. The reverse transcriptase primer as illustrated comprises bead-N2ME-cBC-UMI-poly T segment. In some cases, the reverse transcription and tagmentation step is performed using Tn5N1ME and N2ME-cBC-UMI-T20VN, followed by dual primer PCR (dpPCR) using N1ME/N2ME to amplify only the 3' ends, and then performing an index PCR step. In some cases, cBC is 12-mer and UMI is a 8N mer. In some cases, the UMI is used to barcoded different single cells and transcripts. Sometimes, the N2ME-cBC-UMI-T20VN primers can be generated by split-and-pool cycles of DNA synthesis on beads to obtain 4^12 (about 16.8 million) barcoded beads with reverse transcription primers. In some instances, the reverse transcription primer comprises cBC and UMI and is hybridized on a bead. The reverse transcriptase primer hybridizes to the poly A tail at the 3' end of the mRNA molecule, and the reverse transcriptase synthesizes a cDNA molecule complementary to the mRNA molecule, extending from the reverse transcriptase primer. In some instances, the reverse transcriptase is SuperScript IV reverse transcriptase, and the reverse transcription reaction takes place at 50°C for 30 minutes. The mRNA-cDNA hybrid molecules undergo a tagmentation reaction by a transposome in the same reaction chamber. The transposome comprises a transposase and a nucleic acid molecule comprising a first tag (N1) and a mosaic end segment. Sometimes, the transposome comprises a Tn5-N1ME. The tags are illustrated as gray lines and dotted lines in **FIG. 3**, where dotted line represents the tag comprising N2 and represents a continuous molecule without a break and gray line represent the tag comprising N1, as mentioned above in the context of **FIG. 2**. In the tagmentation reaction, the transposome inserts the N1 tag at the cleaved 5' end of the mRNA-cDNA hybrid to generate a plurality of dual-end tagged mRNA-cDNA hybrid fragments having 5' overhang tags of N1ME at the cleaved 5' ends. The dual-end tagged mRNA-cDNA hybrid fragments comprises a double-stranded mRNA-cDNA fragment of the double-stranded mRNA-cDNA hybrid having a 5' overhang tag extending from the 5' end of mRNA strand and a 5' overhang tag extending from the 5' end of cDNA strand, where the 5' overhang tag comprises N1ME. After the reverse transcription and tagmentation reaction, the transposase is inactivated. In some instances, a protease is introduced to the reaction chamber to inactivate the transposase. In some instances, the protease is active at a temperature of no more than 65°C and can be inactivated by heating to a temperature greater than greater than 65°C after the transposases have been inactivated. Then, the plurality of dual-end tagged mRNA-cDNA hybrid fragments having overhang tags are subject to a gap filling or an extension reaction to generate a plurality of blunt-ended, dual-end tagged mRNA-cDNA hybrid fragments. In the extension reaction, complementary segments to the 5' overhang tags from 3' recessed ends of the mRNA-cDNA hybrid fragments are synthesized to form blunt-ended mRNA-cDNA hybrid fragments. In some cases, the extension reaction utilizes a DNA polymerase to synthesize the complementary segments to the overhang tags. The blunt ended, dual-end tagged mRNA-cDNA hybrid fragments comprises two individual strands, where an individual strand comprises a tag at a 5' end of the fragment, an mRNA or cDNA fragment, and a reverse complement of another tag at a 3' end of the fragment. In some instances, the individual strand comprises N1ME at a 5' end, an mRNA fragment, and a reverse complement of UMI-cBC-MEN2 at a 3' end. In some instances, the individual strand comprises bead-N2ME-cBC-UMI-poly T at a 5' end, a cDNA fragment, and a reverse complement of N1ME at a 3' end. Next, a primer-directed extension reaction, or an amplification reaction, is performed to selectively generate copies of blunt-ended cDNA fragments corresponding to the 3' ends of the mRNA molecules. In some embodiments, the amplification is a dual primer PCR (dpPCR) using primers comprising N1ME and N2ME. In some cases, the dpPCR is followed by an index PCR. In some cases, 16 cycles of index PCR are performed. In some cases, at least 2, 3, 4, 5, 6, 7, 8, 9, 10, 11, 12, 13, 14, 15, 16, 17, 18, 19, 20 cycles of index PCR are performed. After the amplification, the plurality of blunt-ended cDNA fragments comprise individual strands comprising a tag at a 5' end of a cDNA fragment, the cDNA fragment of a 3' end of an mRNA molecule, a reverse complement of another tag at a 3' end of the cDNA fragment, and a sequence adapter at either 5' or 3' end. In some cases, the individual strand comprises a second tag (N2) at a 5' end, the cDNA fragment of a 3' end of an mRNA molecule, and a reverse complement of a first tag at a 3' end (N1'), and a sequence adapter at 3' end of N1'. In some cases, the individual strand comprises a sequence adapter at 5' end, a first tag (N1), the cDNA fragment of a 3' end of an mRNA molecule, and a reverse complement of a second tag (N2') at a 3' end.

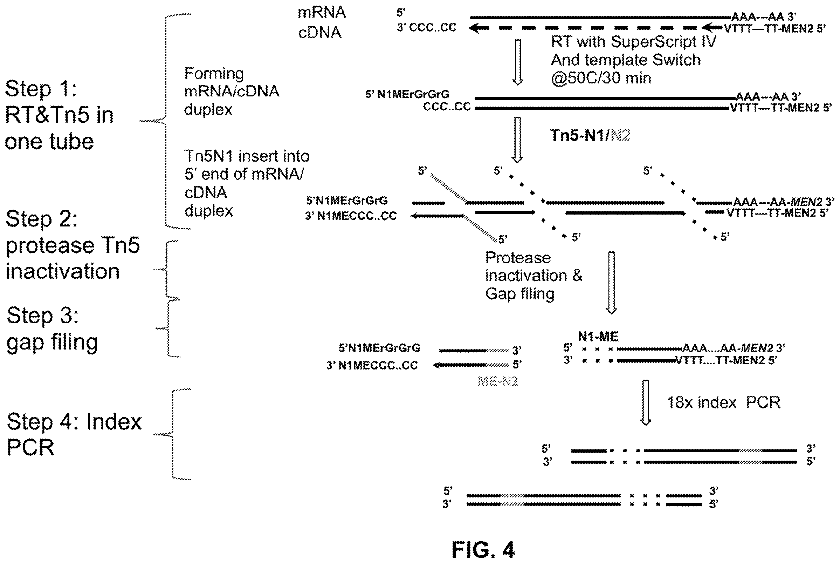

**FIG. 4** illustrates the capability to generate a cDNA library from a bulk of mRNA molecules of greater than 100 ng total RNA by the disclosure provided herein. The bulk mRNA sample is subjected to reverse transcription and tagmentation in a single chamber in a single step. The mRNA molecules are contacted with a reverse transcriptase and a reverse transcriptase primer comprising a second tag (denoted as N2), a mosaic end (denoted as ME), and a poly T segment. In some cases, the reverse transcription and tagmentation step is performed using Tn5N1ME and N2ME- T20VN. The reverse transcriptase primer hybridizes to the poly A tail at the 3' end of the mRNA molecule, and the reverse transcriptase synthesizes a cDNA molecule complementary to the mRNA molecule, extending from the reverse transcriptase primer. In some instances, the reverse transcriptase is SuperScript IV reverse transcriptase, and the reverse transcription reaction takes place at 50°C for 30 minutes. The mRNA-cDNA hybrid molecules also are contacted by a template switching oligonucleotide (TSO). The TSO hybridizes to the 3' end of the cDNA that often comprises a poly-deoxycytidine segment. In some cases, the TSO comprises a poly-guanidine ribonucleotide segment, a ME, and a first tag (N1) at a '5 end. The addition of TSO in the reverse transcription and tagmentation reaction allows for a full length capture of the mRNA molecules. The mRNA-cDNA hybrid molecules having a TSO undergo a tagmentation reaction by a transposome in the same reaction chamber. The transposome comprises a transposase and a first tag (N1) or a second tag (N2). The tags are illustrated as gray lines and dotted lines in **FIG. 4**. Often, the transposome comprises at least one of Tn5-N1, Tn5-N2, or Tn5-N1N2. In the tagmentation reaction, the transposome inserts the first or second tag at the cleaved 5' end of the mRNA-cDNA hybrid to generate a plurality of dual-end tagged mRNA-cDNA hybrid fragments having 5' overhang tags at the cleaved 5' ends. The dual-end tagged mRNA-cDNA hybrid fragments comprises a double-stranded mRNA-cDNA fragment of the double-stranded mRNA-cDNA hybrid having a 5' overhang tag extending from the 5' end of mRNA strand and a 5' overhang tag extending from the 5' end of cDNA strand, where the 5' overhang tag comprises N1 or N2. After the reverse transcription and tagmentation reaction, the transposase is inactivated. In some instances, a protease is introduced to the reaction chamber to inactivate the transposase. In some instances, the protease is active at a temperature of no more than 65°C and can be inactivated by heating to a temperature greater than greater than 65°C after the transposases have been inactivated. Then, the plurality of dual-end tagged mRNA-cDNA hybrid fragments having overhang tags are subject to a gap filling or an extension reaction to generate a plurality of blunt-ended, dual-end tagged mRNA-cDNA hybrid fragments. In the extension reaction, complementary segments to the 5' overhang tags from 3' recessed ends of the mRNA-cDNA hybrid fragments are synthesized to form blunt-ended mRNA-cDNA hybrid fragments. In some cases, the extension reaction utilizes a DNA polymerase to synthesize the complementary segments to the overhang tags. The blunt ended, dual-end tagged mRNA-cDNA hybrid fragments comprises two individual strands, where an individual strand comprises a tag at a 5' end of the fragment, an mRNA or cDNA fragment, and a reverse complement of another tag at a 3' end of the fragment. In some instances, the individual strand comprises a first tag and a ME segment (N1ME) a 5' end, mRNA or cDNA fragment, and a reverse complement of a second tag and a ME segment (N2'ME) at a 3' end. In some instances, the individual strand comprises a second tag and a ME segment (N2ME) a 5' end, mRNA or cDNA fragment, and a reverse complement of a first tag and a ME segment (N1 'ME) at a 3' end. Next, a primer-directed extension reaction, or an amplification reaction, is performed to generate copies of a plurality of blunt-ended cDNA fragments. Use of primers comprising the first tag or a portion thereof or primers comprising the second tag or a portion thereof may allow for the generation of copies of any blunt-ended fragment comprising the first tag or the second tag, respectively. In some cases, the primers comprise sequence adapters. In some cases, 18 cycles of index PCR are performed. After the amplification, the plurality of blunt-ended cDNA fragments comprise individual strands comprising a tag at a 5' end of a cDNA fragment, the cDNA fragment, a reverse complement of another tag at a 3' end of the cDNA fragment, and a sequence adapter at either 5' or 3' end In some cases, the individual strand comprises a first tag (N1) at a 5' end, the cDNA fragment, and a reverse complement of a second tag (N2'), and a sequence adapter at a 3' end. In some cases, the individual strand comprises a sequence adapter at 5' end of a second tag, a second tag (N2), the cDNA fragment, and a reverse complement of a first tag at a 3' end (N1').

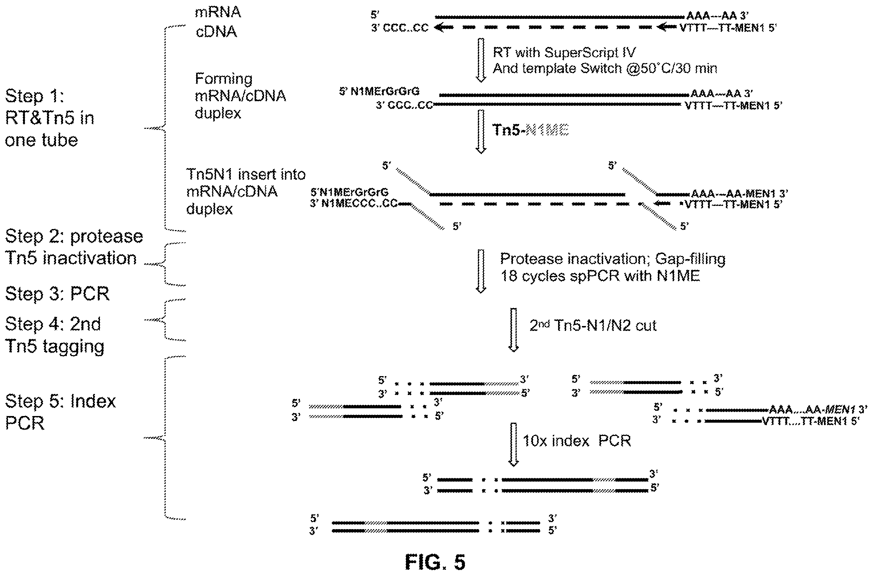

**FIG. 5** illustrates capturing the full length of mRNA and the genomic DNA from a single cell by the disclosure provided herein. As shown in **FIG. 5**, the reverse transcription and tagmentation reaction is followed by a single primer PCR (spPCR) preamplification reaction. The disclosures provided herein allows for at least one of obtaining the mRNA and genomic DNA in single step, a shorter extension time during preamplification, and a full length coverage of mRNA. The mRNA molecules from a single cell are subjected to reverse transcription and tagmentation in a single chamber in a single step. The mRNA molecules are contacted with a reverse transcriptase and a reverse transcriptase primer comprising a first tag (N1), a mosaic end (ME), and a poly T segment. In some cases, the reverse transcriptase primer is N1ME-T20VN. The reverse transcriptase primer hybridizes to the poly A tail at the 3' end of the mRNA molecule, and the reverse transcriptase synthesizes a cDNA molecule complementary to the mRNA molecule, extending from the reverse transcriptase primer. In some instances, the reverse transcriptase is SuperScript IV reverse transcriptase, and the reverse transcription reaction takes place at 50°C for 30 minutes. The mRNA-cDNA hybrid molecules also are contacted by a TSO that hybridizes to the 3' end of the cDNA that often comprises a poly-deoxycytidine segment and links to a 5' end of the mRNA molecules. In some cases, the TSO comprises a poly-guanidine ribonucleotide segment, a ME, and a first tag (N1) at a '5 end. The addition of TSO in the reverse transcription and tagmentation reaction allows for a full length capture of the mRNA molecules. The mRNA-cDNA hybrid molecules having a TSO undergo a tagmentation reaction by a transposome in the same reaction chamber. The transposome comprises a transposase and a nucleic acid molecule comprising N1 and ME. In some cases, the transposome is Tn5-N1ME. In the tagmentation reaction, the transposome inserts the first tag at the cleaved 5' end of the mRNA-cDNA hybrid to generate a plurality of dual-end tagged mRNA-cDNA hybrid fragments having 5'overhang tags comprising N1 at the cleaved 5' ends. The tags are illustrated as gray lines and dotted lines in **FIG. 5**, where a dotted line represents the tag comprising N2 and represents a continuous molecule without a break and gray line represent the tag comprising N1, as mentioned above in the context of **FIG. 2**. After the reverse transcription and tagmentation reaction, the transposase is inactivated. In some instances, a protease is introduced to the reaction chamber to inactivate the transposase. In some instances, the protease is active at a temperature of no more than 65°C and can be inactivated by heating to a temperature greater than greater than 65°C after the transposases have been inactivated. Then, the plurality of dual-end tagged mRNA-cDNA hybrid fragments having overhang tags are subject to a gap filling or an extension reaction to generate a plurality of blunt-ended, dual-end tagged mRNA-cDNA hybrid fragments. In the extension reaction, complementary segments to the 5' overhang tags from 3' recessed ends of the mRNA-cDNA hybrid fragments are synthesized to form blunt-ended mRNA-cDNA hybrid fragments. In some cases, the extension reaction utilizes a DNA polymerase to synthesize the complementary segments to the overhang tags. The blunt ended, dual-end tagged mRNA-cDNA hybrid fragments comprises two individual strands, where an individual strand comprises a N1 tag at a 5' end of the fragment, an mRNA or cDNA fragment, and a reverse complement of N1 tag at a 3' end of the fragment. Next, a primer-directed extension reaction, or an amplification reaction, is performed to generate copies of a plurality of blunt-ended, dual-end tagged cDNA fragments having N1 tags. The amplification is by 18 cycles of spPCR using primers comprising N1ME. Then, the amplification products of blunt-ended, dual-end tagged cDNA fragments having N1 tags are subjected to a second tagmentation reaction by a second transposome comprising Tn5 and a second tag (N2). The second tagmentation results in a plurality of dual-end tagged cDNA fragments having 5' overhang tags comprising N2. Then, the plurality of dual-end tagged cDNA fragments having N2 overhang tags are subject to a gap filling reaction to generate a plurality of blunt-ended, dual-end tagged mRNA-cDNA hybrid fragments having N1 and N2 tags. In some cases, the resulting blunt-ended, dual-end tagged mRNA-cDNA hybrid fragments having N1 and N2 tags comprise an individual strand comprising a first tag and a ME segment (N1ME) a 5' end, a cDNA fragment, and a reverse complement of a second tag and a ME segment (N2'ME) at a 3' end. In some cases, the resulting blunt-ended, dual-end tagged mRNA-cDNA hybrid fragments having N1 and N2 tags comprise an individual strand comprising a second tag and a ME segment (N2ME) a 5' end, a cDNA fragment, and a reverse complement of a first tag and a ME segment (N1'ME) at a 3' end. Next, a primer-directed extension reaction, or an amplification reaction, is performed to generate copies of a plurality of blunt-ended cDNA fragments. In some cases, the primers comprise sequence adapters. In some cases, 10 cycles of index PCR are performed. After the amplification, the plurality of blunt-ended cDNA fragments comprise individual strands comprising a tag at a 5' end of a cDNA fragment, the cDNA fragment, a reverse complement of another tag at a 3' end of the cDNA fragment, and a sequence adapter at either 5' or 3' end. In some cases, the individual strand comprises a first tag (N1) at a 5' end, the cDNA fragment, and a reverse complement of a second tag (N2'), and a sequence adapter at a 3' end. In some cases, the individual strand comprises a sequence adapter at 5' end of a second tag, a second tag (N2), the cDNA fragment, and a reverse complement of a first tag at a 3' end (N1').

Various embodiments described herein offer advantages in generating transposome enabled DNA/RNA-seq (tedRNA-Seq) to generate a cDNA library. The disclosure provided herein allows for creation of dual-end-labeled RNA-DNA hybrid fragment from an mRNA template by using a reverse transcriptase primer and a Tn5-transposome comprising a transposon tag in a single step reaction in a single reaction chamber by using the reaction condition that allow for reverse transcription and Tn5 insertion to function in the same reaction. The reverse transcriptase primer often has a poly T segment. The reverse transcriptase primer sometimes has a gene specific segment. In some cases, the disclosure provides a method to process many individual single cells to capture mRNA with distinct barcoded fragments.

The disclosure herein enables capture and tagging of both mRNA and genomic DNA from a single sample in a single reaction, where the sample can be single cell or bulk lysate from many cells. In some cases, the cell is lysed, and the sample from the lysed cell undergoes reverse transcription and tagmentation in a single step. In some cases, many individual single cells are processed to capture mRNA and genomic DNA with distinct barcoded fragments.

Disclosed herein is a method to combine reverse transcription, second strand synthesis and tagmentation reactions to capture full length mRNA in a single step reaction. In some cases, the method includes TSO in the reaction as primer for second strand synthesis. In some cases, method includes obtaining the full length RNAs from 1k-10k cells by barcoding both reverse transcriptase primer and Tn5's tagmentation transposomes.

The disclosure provided herein offer a transposome enabled DNA/RNA-Seq (tedRNA-Seq) based on OnePot reaction to achieve a) cell lysis, b) reverse transcriptase, c) Tn5 tagmentation, and d) template switch all in one reaction step, then following with libraries index PCR.

Also provided herein is a method that allows for RNA-seq without need to remove gDNA before the reaction. In some cases, the RNA-seq obtains the 3' end segments with polyT as the reverse transcriptase primer. In some cases, the RNA-seq is performed with gene specific primer for region of interest without removing gDNA.

### Reaction Mixtures

The present disclosure also provides reaction mixtures useful for performing any of the subject methods. A reaction mixture for performing a subject method can comprise one or more elements disclosed herein in relation to any of the various aspects or in any combination In an aspect, the present disclosure provides a reaction mixture comprising an mRNA strand from a sample; and a composition comprising (i) a first transposome comprising a transposase and a first transposon nucleic acid, (ii) a reverse transcriptase, and (iii) a reverse transcriptase primer comprising a second transposon nucleic acid. In some instances, the reaction mixture comprises a double-stranded polynucleotide comprising genomic DNA from the sample. In some instances, the reaction mixture comprises a first primer comprising a gene specific sequence and the second transposon sequence, and a second primer comprising a complement of the gene specific sequence and the second transposon sequence. In some instances, the reaction mixture comprises a second transposome comprising a transposase and the second transposon sequence. In some instances, the reaction mixture comprises a reverse transcriptase primer having at least one of a poly-T segment, a complementary segment to a gene of interest, and a cell barcode and a unique molecular identifier (UMI). In some instances, the reaction mixture comprises a reverse transcriptase primer bound to a bead. In some instances, the reaction mixture comprises a template switching oligonucleotide (TSO). In some instances, the TSO comprises a first transposon sequence and a mosaic end. In some instances, the reaction mixture comprises a protease that inactivates the transposase.

### Kit

The present disclosure also provides kits useful for performing any of the subject methods. The kit can comprise one or more elements disclosed herein in relation to any of the various aspects or in any combination. A kit can comprise transposases, for example Tn transposases. A kit can comprise transposon nucleic acids. A kit can comprise at least one enzyme for conducting an extension reaction. A kit can comprise at least one primer for conducting a primer-directed extension reaction. A kit can comprise at least one barcoded primer for conducting a primer-directed extension reaction.

Described herein are kits comprising a transposome having a polypeptide transposase and a nucleic acid comprising a mosaic end and a first tag, a reverse transcriptase, an oligonucleotide comprising a mosaic end and a second tag, and instructions for practicing methods as described herein. In some instances, the oligonucleotide acts as a reverse transcriptase primer.

In some instances, a kit comprises a set of transposomes comprising a homodimer transposase comprising a first transposon sequence, a homodimer transposase comprising a second transposon sequence, and optionally a heterodimer transposase comprising the first transposon sequence and the second transposon sequence; a reverse transcriptase; a reverse transcriptase primer comprising the first transposon sequence; and instructions for practicing method as described herein.

The contents of the kit may be used in any suitable chamber. In some instances, the chamber is a well, a plate, a tube, a chamber, a flow cell, or a chip. In some instances, the chamber is a bead, a droplet, or a defined aqueous space in an emulsion.

The contents of the kit may be contained in any suitable container. Each component may be packaged into different containers or where cross-reactivity and shelf-life permit, combinations of components can be provided in chambers. Non-limiting examples of containers include a well, a plate, a tube, a chamber, a flow cell, or a chip.

The contents of the kit may be immediately usable for performing the methods described herein. In some cases, the contents of the kit are combined with other reagents in the kit or reagents supplied by a user prior to use in methods described herein. For example, a concentrated composition can be diluted prior to use or a lyophilized composition can be reconstituted prior to use.

A kit may provide buffers, non-limiting examples of which include sodium carbonate buffer, a sodium bicarbonate buffer, a borate buffer, a Tris buffer, a MOPS buffer, a HEPES buffer, and combinations thereof. A kit may comprise a control sample, e.g., purified DNA for use as a positive control or quantification standard.

In various embodiments, the kit further comprises nucleotides. The nucleotides can be ribonucleotides, deoxyribonucleotides, synthetic nucleotides or combinations thereof. Often, the nucleotides are deoxyribonucleotides.

In some embodiments, the kit further comprises adapter oligonucleotides. The adapter oligonucleotides can be single-stranded or double-stranded. The adapter may be attached to either a 5' end or a 3' end of a nucleic acid molecule. In some cases, the adapter oligonucleotides comprise a barcode sequence, amplification primer binding sequence, sequencing primer binding sequence, or combinations thereof.

A transposase supplied in a kit can be any transposase described herein, including, but not limited to, integrase, HERMES, or HIV integrase. The transposase can be a Tn transposase (e.g. Tn3, Tn5, Tn7, Tn10, Tn552, Tn903), a MuA transposase, a Vibhar transposase (e.g. from Vibrio harveyi), Ac-Ds, Ascot-1, Bs1, Cin4, Copia, En/Spm, F element, hobo, Hsmarl, Hsmar2, IN (HIV), IS1, IS2, IS3, IS4, IS5, IS6, IS10, IS21, IS30, IS50, IS51, IS150, IS256, IS407, IS427, IS630, IS903, IS911, IS982, IS1031, ISL2, L1, Mariner, P element, Tam3, Tc1, Tc3, Tel, THE-1, Tn/O, TnA, Tol1, Tol2, TnlO, Tyl, any prokaryotic transposase, or any transposase related to and/or derived from those listed above. In some embodiments, the transposase supplied in the kit is a Tn transposase, an MuA transposase, or a Vibhar transposase. In some cases, the transposase supplied in the kit is a Tn transposase selected from Tn3, Tn5, Tn7, and Tn10. In some cases, the transposomes comprise a dimer of monomers, individual monomers comprising a transposase and a transposon nucleic acid. The transposome dimer can be a homodimer or a heterodimer. In some cases, the kit comprises one or more cations to facilitate and/or trigger a transposition reaction. The cations can be divalent cations such as, for example, Ca²⁺, Mg²⁺ and Mn²⁺.

A transposon nucleic acid supplied in a kit can be joined to a nucleic acid molecule. A transposon nucleic acid supplied in a kit can be a single-stranded, a double-stranded or a partially double-stranded nucleic acid molecule sequence. In some cases, the transposon nucleic acid can be either a double-stranded sequence or a partially double stranded sequence. A transposon nucleic acid supplied in a kit can comprise any nucleic acid or nucleic acid analogue suitable for forming a functional complex with a transposase. The transposon nucleic acid can comprise DNA, RNA, modified bases, non-natural bases, a modified backbone, or can comprise nicks in one or both strands.

Kits disclosed herein variously have any reverse transcriptase disclosed herein. In some cases, the kit further comprises a primer to initiate the reverse transcription to synthesize a cDNA molecule. In some instances, the primer comprises a poly T segment. In some cases, the kit further comprises dNTPs. In some embodiments, the kit further comprises a reaction buffer. In some cases, the reaction buffer comprises divalent metal ions. The divalent metal ions often are Mg2+ or Mn2+.

The kit provided herein may optionally use a TSO to provide a full coverage of the mRNA molecule in the resulting cDNA library. The TSO hybridizes to the 3' end of the cDNA that often comprises a poly-deoxycytidine segment and links to a 5' end of the mRNA molecules. In some cases, the TSO comprises a poly-guanidine ribonucleotide segment. In some cases, the TSO further comprises a mosaic end and a first tag (N1) at a `5 end. In some cases, the TSO comprises a mosaic end and a second tag (N2) at a '5 end. The mRNA-cDNA hybrid molecules having a TSO undergo a tagmentation reaction by a transposome in the same reaction chamber.

In some cases, the kit has a protease for inactivating the transposase. In some instances, the protease is a heat-labile protease. Alternatively or in combination, the kit can include an SDS solution for inactivating the transposase. For example, SDS solutions with 0.1%, 0.2% or more SDS can be included in the kit.

A hot-start polymerase supplied in a kit can be used for an extension reaction and/or amplification reaction. Hot-start polymerases, such as high-fidelity PCR polymerases, can be activated by incubation at an elevated temperature for sufficient length of time (e.g., 95 degrees for 1 min).

### System

In an aspect, the present disclosure provides a system for performing methods disclosed herein. The system can comprise (a) a computer configured to receive a user request to characterize a nucleic acid molecule, or generate a nucleic acid library; (b) one or more processors configured to execute commands that effect a reverse transcription and tagmentation reaction in a single chamber and one or more extension reactions (including primer-directed extension reactions) on a nucleic acid molecule or a portion thereof in response to the user request; and/or (c) a sequencing system capable of generating sequence reads. In some embodiments, the computer comprises one or more processors. Processors may be associated with one or more controllers, calculation units, and/or other units of a computer system, or implanted in firmware as desired. If implemented in software, the routines may be stored in any computer readable memory such as in a hard drive, a solid-state memory drive, RAM, ROM, flash memory, a magnetic disk, a laser disk, or other storage medium. In some instances, the computer readable memory is provided in a cloud or a remote server. Likewise, this software may be delivered to a computing device via any known delivery method including, for example, over a communication channel such as a telephone line, the internet, a wireless connection, etc., or via a transportable medium, such as a computer readable disk, flash drive, etc. The various steps may be implemented as various blocks, operations, tools, modules or techniques which, in turn, may be implemented in hardware, firmware, software, or any combination thereof. When implemented in hardware, some or all of the blocks, operations, techniques, etc. may be implemented in, for example, a custom integrated circuit (IC), an application specific integrated circuit (ASIC), a field programmable logic array (FPGA), a programmable logic array (PLA), etc. In some embodiments, the computer is configured to receive a customer request to design primers for amplifying a specified target sequence (which may also be provided by the customer). The computer may receive the customer request directly (e.g. by way of an input device such as a keyboard, mouse, touch screen operated by the customer or a user entering a customer request, or a voice command given by a user) or indirectly (e.g. through a wired or wireless connection, including over the internet).

In some embodiments, the system comprises a report generator that sends a report to a recipient, wherein the report contains information about a nucleic acid molecule in a sample. For example, the report may contain haplotype information or identification of a structural variation in a nucleic acid molecule. The report may contain information about a low frequency allele or sequence variant. The report may contain information about a subset of regions of a nucleic acid molecule that was selectively sampled. The report generator may send a report automatically in response to the customer request. Alternatively, the report generator may send a report in response to instructions from an operator. The report may be transmitted to a recipient at a local or remote location using any suitable communication medium. For example, the communication medium can be a network connection, a wireless connection, or an internet connection. A report can be transmitted over such networks or connections (or any other suitable means for transmitting information, including but not limited to mailing a physical report, such as a print-out) for reception and/or for review by a recipient. The recipient can be but is not limited to the customer, or electronic system (e.g. one or more computers, and/or one or more servers). In some embodiments, the report generator sends the report to a recipient's device, such as a personal computer, phone, tablet, or other device. The report may be viewed online, saved on the recipient's device, or printed.

In an aspect, the disclosure provides a computer-readable medium comprising codes that, upon execution by one or more processors, implements a method according to any of the methods disclosed herein. Computer readable medium may take many forms, including but not limited to, a tangible storage medium, a carrier wave medium, or physical transmission medium. Non-volatile storage media include, for example, optical or magnetic disks, such as any of the storage devices and/or memory device in any computer(s) or the like, such as may be used to implement the extension reaction and/or amplification reaction, etc. The storage and/or memory device is one or more physical apparatuses used to store data or programs on a temporary or permanent basis. In some embodiments, the device is non-volatile memory and retains stored information when the digital processing device is not powered. In further embodiments, the non-volatile memory comprises flash memory. In some embodiments, the non-volatile memory comprises dynamic random-access memory (DRAM). In some embodiments, the non-volatile memory comprises ferroelectric random access memory (FRAM). In some embodiments, the non-volatile memory comprises phase-change random access memory (PRAM). In other embodiments, the device is a storage device including, by way of non-limiting examples, CD-ROMs, DVDs, flash memory devices, magnetic disk drives, magnetic tapes drives, optical disk drives, punch cards paper tape, any other physical storage medium with patterns of holes, a RAM, a PROM and EPROM, a FLASH-EPROM, any other memory chip or cartridge, a carrier wave transporting data or instructions, cables or links transporting such a carrier wave, or any other medium from which a computer can read programming code and/or data, and cloud computing based storage. In further embodiments, the storage and/or memory device is a combination of devices such as those disclosed herein. In some embodiments, the device is volatile memory and requires power to maintain stored information. Volatile storage media include dynamic memory, such as main memory of a computer. Tangible transmission media include coaxial cables; copper wire and fiber optics, including the wires that comprise a bus within a computer system. Carrier-wave transmission media can take the form of electric or electromagnetic signals, or acoustic or light waves such as those generated during radio frequency (RF) and infrared (IR) data communications. Many of these forms of computer readable media may be involved in carrying one or more sequences of one or more instructions to a processor for execution.

### Definitions

The practice of some methods disclosed herein employ, unless otherwise indicated, conventional techniques of immunology, biochemistry, chemistry, molecular biology, microbiology, cell biology, genomics and recombinant DNA, which are within the skill of the art. See for example Sambrook and Green, Molecular Cloning: A Laboratory Manual, 4th Edition (2012); the series Current Protocols in Molecular Biology (F. M. Ausubel, et al. eds.); the series Methods In Enzymology (Academic Press, Inc.), PCR 2: A Practical Approach (M.J. MacPherson, B.D. Hames and G.R. Taylor eds. (1995)), Harlow and Lane, eds. (1988) Antibodies, A Laboratory Manual, and Culture of Animal Cells: A Manual of Basic Technique and Specialized Applications, 6th Edition (R.I. Freshney, ed. (2010)).

The terms "about" and "approximately" mean within an acceptable error range for the particular value as determined by one of ordinary skill in the art, which will depend in part on how the value is measured or determined, such as the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated, the term "about," meaning within an acceptable error range for the particular value, should be assumed.

The terms "polynucleotide", "nucleic acid," and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA (tRNA), ribosomal RNA (rRNA), short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

The term "strand," as used herein, refers to a nucleic acid made up of nucleotides covalently linked together by covalent bonds, e.g., phosphodiester bonds. In a cell, DNA usually exists in a double-stranded form, and as such, has two complementary strands of nucleic acid referred to herein as the "top" and "bottom" strands. In certain cases, complementary strands of a chromosomal region may be referred to as "plus" and "minus" strands, the "first" and "second" strands, the "coding" and "noncoding" strands, the "Watson" and "Crick" strands, or the "sense" and "antisense" strands. The assignment of a strand as being a top or bottom strand is arbitrary and does not imply any particular orientation, function or structure.

A polynucleotide may have a 5' end and 3' end, referring to the end-to-end chemical orientation of a single strand of polynucleotide or nucleic acid. In a single strand of linear DNA or RNA, the chemical convention of naming carbon atoms in the nucleotide sugar-ring means that there generally exists a 5' end which frequently contains a phosphate group attached to the 5' carbon and a 3' end which typically is unmodified from the ribose -OH substituent (hydroxyl group). In some cases, a polynucleotide may have a -OH substituent or a hydroxyl group at a 5' end and -P group or phosphate group at a 3' end. A phosphate group attached to the 5'-end permits ligation of two nucleotides, e.g., the covalent binding of a 5'-phosphate to the 3'-hydroxyl group of another nucleotide, to form a phosphodiester bond. Removal of the 5'-phosphate may inhibit or prevent ligation. The 3'-hydroxyl group is also important as it is joined to the 5'-phosphate in ligation.

The term "tag," as used herein, refers to a nucleic acid molecule that provides a means of identifying a polynucleotide to which it is attached. For example, a tag can comprise a polynucleotide sequence that permits identification, recognition, and/or molecular or biochemical manipulation of the target polynucleotide to which it is attached (e.g., by providing a site for annealing an oligonucleotide, such as a primer for extension by a DNA polymerase, or an oligonucleotide for capture or for a ligation reaction). The process of attaching the tag to a polynucleotide molecule is sometimes referred to herein as "tagging" and a polynucleotide that undergoes tagging or that contains a tag is referred to as "tagged" (e.g., "tagged polynucleotide" or "tagged fragment"). For example, a tagged polynucleotide fragment (e.g., tagged fragment) can comprise a transposon having a transposon nucleic acid as a tag.

The term "wild-type" when made in reference to an allele or sequence, generally refers to the allele or sequence that encodes the phenotype most common in a particular natural population. In some cases, a wild-type allele can refer to an allele present at highest frequency in the population. In some cases, a wild-type allele or sequence refers to an allele or sequence associated with a normal state relative to an abnormal state, for example a disease state.

The terms "mutant" or "variant," when made in reference to an allele or sequence, generally refer to an allele or sequence that does not encode the phenotype most common in a particular natural population. In some cases, a mutant allele can refer to an allele present at a lower frequency in a population relative to the wild-type allele. In some cases, a mutant allele or sequence can refer to an allele or sequence mutated from a wild-type sequence to a mutant sequence that presents a phenotype associated with a disease state. Mutant alleles and sequences may be different from wild-type alleles and sequences by only one base, but can be different up to several bases. The term mutant when made in reference to a gene generally refers to one or more sequence mutations in the gene, including a point mutation, a single nucleotide polymorphism (SNP), an insertion, a deletion, a substitution, a transposition, a translocation, a copy number variation, another genetic mutation, alternation, or sequence variation.

The terms "hybridize," "hybridization," "hybridizing," "anneal," and "annealing," as used herein, generally refer to a reaction in which one or more nucleic acid molecules react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of a PCR, or the enzymatic cleavage of a nucleic acid molecule by a ribozyme. A first sequence that can be stabilized via hydrogen bonding with the bases of the nucleotide residues of a second sequence is said to be "hybridizable" to the second sequence. In such a case, the second sequence can also be said to be hybridizable to the first sequence.

The terms "complement," "complements," "complementary," and "complementarity," as used herein, generally refer to a sequence that is fully complementary to and hybridizable to the given sequence In some cases, a sequence hybridized with a given nucleic acid is referred to as the "complement" or "reverse-complement" of the given molecule if its sequence of bases over a given region is capable of complementarily binding those of its binding partner, such that, for example, A-T, A-U, G-C, and G-U base pairs are formed. In general, a first sequence that is hybridizable to a second sequence is specifically or selectively hybridizable to the second sequence, such that hybridization to the second sequence or set of second sequences is preferred (e.g. thermodynamically more stable under a given set of conditions, such as stringent conditions commonly used in the art) to hybridization with non-target sequences during a hybridization reaction. Typically, hybridizable sequences share a degree of sequence complementarity over all or a portion of their respective lengths, such as between 25%-100% complementarity, including at least 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, and 100% sequence complementarity. Sequence identity, such as for the purpose of assessing percent complementarity, may be measured by any suitable alignment algorithm, including but not limited to the Needleman-Wunsch algorithm (see e.g. the EMBOSS Needle aligner available at
www.ebi.ac.uk/Tools/psa/emboss_needle/nucleotide.html, optionally with default settings), the BLAST algorithm (see e.g. the BLAST alignment tool available at
blast.ncbi.nlm.nih.gov/Blast.cgi, optionally with default settings), or the Smith-Waterman algorithm (see e.g. the EMBOSS Water aligner available at
www.ebi.ac.uk/Tools/psa/emboss_water/nucleotide.html, optionally with default settings). Optimal alignment may be assessed using any suitable parameters of a chosen algorithm, including default parameters.

The term "overhang fragment," as used herein, refers to a double-stranded nucleic acid molecule, e.g., a fragment of a longer double-stranded nucleic acid molecule, having unpaired nucleotides present at one or both ends of the nucleic acid molecule. In cases where an overhang is not present, the double-stranded nucleic acid molecule may be referred to as having a "blunt-end".

The terms "stem-loop product" and "stem-loop structure", as used herein, generally refer to a secondary structure of a nucleic acid molecule in which intramolecular hybridization occurs between portions of the nucleic acid molecule. A stem loop may form when two regions of a single nucleic acid molecule strand hybridize to form a double-stranded portion, which can be referred to as a "stem," and a single-stranded loop that is unpaired, which can be referred to as a "loop". The stem can be of any variable length of base pairs, and base pairing along a stem may be interrupted internally by gaps of one or more unpaired bases on one or both portions participating in the stem. The loop can be of any variable length of unpaired bases. In some cases, the loop is at least 3 bases in length. In some cases, the two regions forming the "stem" are completely complementary. In some cases, the two regions forming the "stem" are partially complementary. In some cases, a single nucleic acid molecule may comprise one stem loop structure. In some cases, a single nucleic acid molecule may comprise more than one stem loop structure. The stem portion of a stem loop structure may terminate as a double stranded section with no overhangs, with a single stranded section comprising a 5' overhang, with a single stranded section comprising a 3' overhang, or with single-stranded portions extending from both the 5' end and the 3' end. A stem loop structure can also be referred to as a "hairpin" or "hairpin structure."

The terms "adaptor" or "adapter," as used herein, generally refer to a nucleic acid which can be attached to another nucleic acid molecule. For example, an adaptor can refer to a single-stranded nucleic acid molecule which can be attached to a single-stranded nucleic acid molecule (e.g., a cell-free nucleic acid molecule, fragment of a cell-free nucleic acid molecule, genomic DNA, or fragment of genomic DNA). In some cases, an adaptor can refer to a double-stranded nucleic acid which can be attached to another double-stranded nucleic acid (e.g., a cell-free nucleic acid molecule, fragment of a cell-free nucleic acid molecule, genomic DNA, or fragment of genomic DNA). An adaptor can be attached to either a 5' end or a 3' end of a nucleic acid molecule. In some cases, an adaptor can be attached to both ends of a nucleic acid molecule, that is, one adaptor to each end.

The term "primer," as used herein, generally refers to an oligonucleotide, either natural or synthetic, that is capable, upon forming a duplex with a nucleic acid molecule template, of acting as a point of initiation of nucleic acid synthesis and being extended from its 3' end along the template so that an extended duplex is formed. The sequence of nucleotides added during the extension reaction may be determined by the sequence of the template nucleic acid molecule. Usually primers are extended by a DNA polymerase. Sometimes primers are extended by a reverse transcriptase. Primers are generally of a length compatible with their use in synthesis of primer extension products, and usually are in the range of between 8 to 100 nucleotides in length, such as 10 to 75, 15 to 60, 15 to 40, 18 to 30, 20 to 40, 21 to 50, 22 to 45, 25 to 40, and so on, more typically in the range of between 18-40, 20-35, 21-30 nucleotides long, and any length between the stated ranges. Typical primers can be in the range of between 10-50 nucleotides long, such as 15-45, 18-40, 20-30, 21-25 and so on, and any length between the stated ranges. In some embodiments, the primers are usually not more than about 10, 12, 15, 20, 21, 22, 23, 24, 25, 26, 27, 28, 29, 30, 35, 40, 45, 50, 55, 60, 65, or 70 nucleotides in length.

Primers are usually single-stranded for maximum efficiency in amplification, but may alternatively be double-stranded. If double-stranded, the primers can first be treated to separate its strands before being used to prepare primer extension products, or simply referred to as extension products. This denaturation step can be effectuated by heat, but may alternatively be carried out using alkali, followed by neutralization. Thus, a "primer" is complementary to a nucleic acid molecule template, and complexes by hydrogen bonding or hybridization with the template to give a primer/template complex for initiation of synthesis by a polymerase, which is extended by the addition of covalently bonded bases linked at its 3' end complementary to the template in the process of DNA synthesis.

The term "extension product," as used herein, generally refers to a product of a reaction in which a nucleotide primer is extended by the covalent addition of nucleotides. In some cases, the nucleotide incorporation can be guided by a template. In some cases, the nucleotide incorporation can occur without a template (e.g., "template-independent"). In some cases, an extension product is an amplification product, such as from PCR amplification, rolling circle amplification (RCA), or isothermal amplification.

The terms "amplify," "amplifies," "amplified," and "amplification," as used herein, generally refer to any process by which one or more copies are made of a target nucleic acid molecule or a portion thereof. A variety of methods of amplifying nucleic acid molecules (e.g. DNA and/or RNA) are available, some examples of which are described herein. Amplification may be linear, exponential, or involve both linear and exponential phases in a multi-phase amplification process. Amplification methods may involve changes in temperature, such as a heat denaturation step, or may be isothermal processes that do not require heat denaturation. In some cases, the amplification is effected by means of PCR using a pair of primers. Amplified products can be subjected to subsequent analyses, including but not limited to melting curve analysis, nucleotide sequencing, single-strand conformation polymorphism assay, allele-specific oligonucleotide hybridization, Southern blot analysis, and restriction endonuclease digestion.

The terms "isolated" and "isolating," with reference to a nucleic acid molecule or nucleic acid molecule complex, including but not limited to tagmentation products and extension products, generally refer to a preparation of the substance (e.g., nucleic acid molecule, nucleic acid molecule complex, tagmentation products and extension products thereof) devoid of at least some of the other components that may also be present where the substance or a similar substance naturally occurs or is initially obtained from (e.g., a biological sample, a sample reaction volume, e.g., a tagmentation reaction volume, an extension reaction volume, etc). For example, an isolated substance may be prepared using a purification technique to enrich it from a source mixture Enrichment can be measured on an absolute basis or in terms of a concentration, for example in terms of weight per volume of solution, molecules per volume of solution, or any other appropriate measure.

The terms "RNA-DNA," "DNA-RNA," "RNA-DNA hybrid," or "DNA-RNA hybrid," as use herein, generally refers to a nucleic acid molecule comprising ribonucleotides and deoxyribonucleotides. In some instances, the RNA-DNA has a strand of ribonucleotides and a strand of deoxyribonucleotides where the deoxyribonucleotide strand is complementary to the ribonucleotide strand. Often, the RNA-DNA is a RNA strand and a DNA strand that is complementary to the RNA strand. Sometimes the RNA-DNA has a strand that comprises both ribonucleotides and deoxyribonucleotides.

### EXAMPLES

The following examples are given for the purpose of illustrating various embodiments of the disclosure and are not meant to limit the present disclosure in any fashion. The present examples, along with the methods described herein, are examples and are not intended as limitations on the scope of the disclosure.

### Example 1

Provided herein is an exemplary method of producing a cDNA library of an mRNA molecule from dual-end tagged RNA-DNA hybrid fragments.

To generate N1-ME containing transposome complexes, equal molar DNA oligos transposon N1-ME: GTAGGTGTGAGTGATGGTTGAGGTAGT-AGATGTGTATAAGAGACAG (SEQ ID NO: 1) and ME': (5phos)-CTGTCTCTTATACACATCT (SEQ ID NO: 2) were annealed in the presence of 1x STE (10 mM Tris pH 8.0, 50 mM NaCl, 1 mM EDTA) by incubating at 95°C for 1 minute and then cooling down to room temperature. Double-stranded transposon was then incubated with Tn5 transposase at 1.2:1 molar ratio at room temperature for 30 minutes to form transposome complexes (Tn5-N1-ME).

To generate N2-ME containing transposome complexes, equal molar DNA oligos transposon N2-ME: GTGAGTGATGGTTGAGGTAGTGTGGAG-AGATGTGTATAAGAGACAG (SEQ ID NO: 3) and ME': (5phos)-CTGTCTCTTATACACATCT (SEQ ID NO: 2) were annealed in the presence of 1x STE (10 mM Tris pH 8.0, 50 mM NaCl, 1 mM EDTA) by incubating at 95°C for 1 minute then cooling down to room temperature. Double-stranded transposon was then incubated with Tn5 transposase at 1.2:1 molar ratio at room temperature for 30 minutes to form transposome complexes (Tn5-N2-ME).

30 pg mRNA was incubated with SuperScript IV reverse transcriptase, reverse transcriptase primer of N1ME-T20VN, dNTPs, transposome complex Tn5-N1-ME, and transposome complex Tn5-N2-ME in the presence of 5 mM MgCh at 55°C for 30 minutes. 1 uL of 2 mg/ml protease was later added to the reaction. The reaction was then incubated at 50°C for 10 minutes, and then at 72°C for 15 minutes.

In indexing amplification, fragmented double-strand mRNA-cDNA hybrid was incubated in a 20 uL reaction with 1 x Q5 Reaction Buffer, 0.02 U/uL Q5 Hot Start High-Fidelity DNA Polymerase (New England BioLabs Inc), 200 uM dNTPs, 0.5 uM indexing primer mix. The reaction was subjected to the following conditions: 72°C for 3 minutes; 98 °C for 2 minutes, followed by 16 cycles of 98°C for 15 seconds and 72 °C for 3 minutes 30 seconds.

The resulting amplified product is purified to serve as library for sequencing on Illumina HiSeq X or NovaSeq sequencer for paired-end reads.

While preferred embodiments of the present disclosure have been shown and described herein, it will be obvious to those skilled in the art that such embodiments are provided by way of example only.

## Claims

1. Use of a composition for tagging of a ribonucleic acid/deoxyribonucleic acid (RNA/DNA) hybrid molecule, the composition comprising a transposome having a polypeptide transposase and a nucleic acid comprising a mosaic end and a first tag, a reverse transcriptase, an oligonucleotide comprising a mosaic end and a second tag, and a RNA/DNA hybrid molecule.

2. The use of claim 1, wherein the first tag and the second tag share a common nucleic acid sequence.

3. The use of claim 1, wherein the first tag and the second tag comprise distinct nucleic acid sequences.

4. The use of any of the proceeding claims, comprising a second transposome having the polypeptide transposase and a second nucleic acid comprising the mosaic end and the second tag.

5. The use of claim 1, wherein the nucleic acid of the transposome comprises a tag that identifies a reaction batch, wherein the tag comprises a UMI.

6. The use of any or the proceeding claims, wherein the oligonucleotide comprises a poly-T segment.

7. The use of any of the proceeding claims, comprising a template switching oligonucleotide (TSO).

8. The use of claim 7, wherein the TSO comprises the first tag and the mosaic end.

9. The use of any of the proceeding claims, comprising a protease that is active at a temperature no more than 65°C and inactive at a temperature greater than 65°C.

10. The use of any of the proceeding claims, comprising an oligonucleotide comprising a complementary segment to a portion of the first tag or the second tag.

11. The use of claim 1, wherein the transposase is a Tn transposase, an MuA transposase, or a Vibhar transposase.

12. The use of any of the proceeding claims1, wherein the RNA/DNA hybrid molecule comprises a dual-end tagged RNA/DNA hybrid molecule comprising 5' overhang tags at 5' ends, wherein the individual 5' overhang tag comprises the first tag or the second tag.

13. The use of any of the proceeding claims, wherein the RNA/DNA hybrid molecule comprises a blunt ended DNA/RNA hybrid molecule comprising complementary segments to the 5' overhang tags from 3' recessed ends of the dual-end tagged RNA/DNA hybrid molecule.

14. The use of claim 13, wherein the blunt ended DNA/RNA hybrid molecules comprises sequence adapters at ends of the blunt-ended DNA/RNA hybrid molecules.

15. The use of claim 13, comprising a sequence library comprising a plurality of blunt ended DNA molecules comprising sequence adapters at ends of the blunt-ended DNA molecules.

## Patentansprüche

1. Verwendung einer Zusammensetzung zum Markieren eines Ribonukleinsäure/Desoxyribonukleinsäure-Hybridmoleküls (RNA/DNA) Hybridmoleküls, wobei die Zusammensetzung ein Transposom mit einer Polypeptidtransposase und einer Nukleinsäure, die ein Mosaikende und eine erste Markierung umfasst, eine reverse Transkriptase, ein Oligonukleotid, das ein Mosaikende und eine zweite Markierung umfasst, und ein RNA/DNA-Hybridmolekül umfasst.

2. Verwendung nach Anspruch 1, wobei die erste Markierung und die zweite Markierung eine gemeinsame Nukleinsäuresequenz teilen.

3. Verwendung nach Anspruch 1, wobei die erste Markierung und die zweite Markierung unterschiedliche Nukleinsäuresequenzen umfassen.

4. Verwendung nach einem der vorstehenden Ansprüche, umfassend ein zweites Transposom mit der Polypeptidtransposase und einer zweiten Nukleinsäure, die das Mosaikende und die zweite Markierung umfasst.

5. Verwendung nach Anspruch 1, wobei die Nukleinsäure des Transposoms eine Markierung umfasst, die einen Reaktionsansatz identifiziert, wobei die Markierung einen UMI umfasst.

6. Verwendung nach einem der vorstehenden Ansprüche, wobei das Oligonukleotid ein Poly-T-Segment umfasst.

7. Verwendung nach einem der vorstehenden Ansprüche, umfassend ein Templatwechsel-Oligonukleotid (TSO).

8. Verwendung nach Anspruch 7, wobei das TSO die erste Markierung und das Mosaikende umfasst.

9. Verwendung nach einem der vorstehenden Ansprüche, umfassend eine Protease, die bei einer Temperatur nicht mehr als 65 °C aktiv ist und bei einer Temperatur höher als 65 °C inaktiv ist.

10. Verwendung nach einem der vorstehenden Ansprüche, umfassend ein Oligonukleotid, das ein zu einem Abschnitt der ersten Markierung oder der zweiten Markierung komplementäres Segment umfasst.

11. Verwendung nach Anspruch 1, wobei die Transposase eine Tn-Transposase, eine MuA-Transposase oder eine Vibhar-Transposase ist.

12. Verwendung nach einem der vorstehenden Ansprüche 1, wobei das RNA/DNA-Hybridmolekül ein doppelendig markiertes RNA/DNA-Hybridmolekül umfasst, das 5'-Überhangmarkierungen an 5'-Enden umfasst, wobei die individuelle 5'-Überhangmarkierung die erste Markierung oder die zweite Markierung umfasst.

13. Verwendung nach einem der vorstehenden Ansprüche, wobei das RNA/DNA-Hybridmolekül ein glattendiges DNA/RNA-Hybridmolekül umfasst, das zu den 5'-Überhangmarkierungen von vertieften 3'-Enden des doppelendig markierten RNA/DNA-Hybridmoleküls komplementäre Segmente umfasst.

14. Verwendung nach Anspruch 13, wobei das glattendige DNA/RNA-Hybridmolekül an Enden des glattendigen DNA/RNA-Hybridmoleküls Sequenzadapter umfasst.

15. Verwendung nach Anspruch 13, umfassend eine Sequenzbibliothek, die eine Vielzahl von glattendigen DNA-Molekülen umfasst, die an Enden der glattendigen DNA-Moleküle Sequenzadapter umfassen.

## Revendications

1. Utilisation d'une composition pour l'étiquetage d'une molécule hybride acide ribonucléique/acide désoxyribonucléique (ARN/ADN), la composition comprenant un transposome ayant un polypeptide transposase et un acide nucléique comprenant une extrémité mosaïque et une première étiquette, une transcriptase inverse, un oligonucléotide comprenant une extrémité mosaïque et une seconde étiquette, et une molécule hybride ARN/ADN.

2. Utilisation de la revendication 1, dans laquelle la première étiquette et la seconde étiquette partagent une séquence d'acide nucléique commune.

3. Utilisation de la revendication 1, dans laquelle la première étiquette et la seconde étiquette comprennent des séquences d'acide nucléique distinctes.

4. Utilisation de l'une quelconque des revendications précédentes, comprenant un second transposome ayant le polypeptide transposase et un second acide nucléique comprenant l'extrémité mosaïque et la deuxième étiquette.

5. Utilisation de la revendication 1, dans laquelle l'acide nucléique du transposome comprend une étiquette qui identifie un lot de réaction, l'étiquette comprenant un UMI.

6. Utilisation de l'une quelconque des revendications précédentes, dans laquelle l'oligonucléotide comprend un segment poly-T.

7. Utilisation de l'une quelconque des revendications précédentes, comprenant un oligonucléotide de commutation de matrice (TSO).

8. Utilisation de la revendication 7, dans laquelle le TSO comprend la première étiquette et l'extrémité en mosaïque.

9. Utilisation de l'une quelconque des revendications précédentes, comprenant une protéase qui est active à une température ne dépassant pas 65 °C et inactive à une température supérieure à 65 °C.

10. Utilisation de l'une quelconque des revendications précédentes, comprenant un oligonucléotide comprenant un segment complémentaire à une partie de la première étiquette ou de la seconde étiquette.

11. Utilisation de la revendication 1, dans laquelle la transposase est une transposase Tn, une transposase MuA ou une transposase Vibhar.

12. Utilisation de l'une quelconque des revendications précédentes 1, dans laquelle la molécule hybride ARN/ADN comprend une molécule hybride ARN/ADN étiquetée aux deux extrémités comprenant des étiquettes en surplomb 5' aux extrémités 5', l'étiquette en surplomb 5' individuelle comprenant la première étiquette ou la seconde étiquette.

13. Utilisation de l'une quelconque des revendications précédentes, dans laquelle la molécule hybride ARN/ADN comprend une molécule hybride ADN/ARN à extrémité franche comprenant des segments complémentaires aux étiquettes en surplomb 5' des extrémités en retrait 3' de la molécule hybride ARN/ADN à étiquette double.

14. Utilisation de la revendication 13, dans laquelle les molécules hybrides ADN/ARN à extrémités franches comprennent des adaptateurs de séquence aux extrémités des molécules hybrides ADN/ARN à extrémités franches.

15. Utilisation de la revendication 13, comprenant une bibliothèque de séquences comprenant une pluralité de molécules d'ADN à extrémités franches comprenant des adaptateurs de séquence aux extrémités des molécules d'ADN à extrémités franches.
